# EUROPEAN PATENT SPECIFICATION

(11) **EP 3 840 827 B1**
(45) Date of publication and mention of the grant of the patent: **01.04.2026**
(21) Application number: 19852675.8
(22) Date of filing: 23.08.2019
(51) Int. Cl.: A61N 1/05, A61N 1/36, A61M 5/142, A61M 5/152, A61M 39/02

(54) **MASS TRANSPORT INSIDE MAMMALS**
MASSENTRANSPORT IN SÄUGETIEREN
TRANSPORT DE MASSE À L'INTÉRIEUR DE MAMMIFÈRES

(30) Priority: 24.08.2018 US 201862722273 P
(43) Date of publication of application: 30.06.2021
(73) Proprietor: Cochlear Limited, Macquarie University, New South Wales 2109 (AU)
(72) Inventor: SMYTH, Daniel, Macquarie University, New South Wales 2109 (AU); MURPHY, Richard Bruce, Macquarie University, New South Wales 2109 (AU)
(74) Representative: Grünecker Patent- und Rechtsanwälte PartG mbB
(86) International application number: PCT/IB2019/057123
(87) International publication number: WO 2020/039402

(56) References cited:
- WO-A1-2018/027102
- US-A1- 2004 078 057
- US-A1- 2010 030 130
- US-A1- 2010 121 256
- US-A1- 2010 121 256
- US-A1- 2014 018 736
- US-A1- 2017 028 182
- US-A1- 2017 028 182
- US-B1- 6 685 697
- US-B2- 7 650 194

## Description

### BACKGROUND

Hearing loss, which may be due to many different causes, is generally of two types: conductive and sensorineural. Sensorineural hearing loss is due to the absence or destruction of the hair cells in the cochlea that transduce sound signals into nerve impulses. Various hearing prostheses are commercially available to provide individuals suffering from sensorineural hearing loss with the ability to perceive sound. One example of a hearing prosthesis is a cochlear implant.

Conductive hearing loss occurs when the normal mechanical pathways that provide sound to hair cells in the cochlea are impeded, for example, by damage to the ossicular chain or the ear canal. Individuals suffering from conductive hearing loss may retain some form of residual hearing because the hair cells in the cochlea may remain undamaged.

Individuals suffering from conductive hearing loss typically receive an acoustic hearing aid. Hearing aids rely on principles of air conduction to transmit acoustic signals to the cochlea. In particular, a hearing aid typically uses an arrangement positioned in the recipient's ear canal or on the outer ear to amplify a sound received by the outer ear of the recipient. This amplified sound reaches the cochlea causing motion of the perilymph and stimulation of the auditory nerve.

In contrast to hearing aids, which rely primarily on the principles of air conduction, certain types of hearing prostheses commonly referred to as cochlear implants convert a received sound into electrical stimulation. The electrical stimulation is applied to the cochlea, which results in the perception of the received sound.
Implantable drug delivery systems according to the state of the art are disclosed in the US patent applications US 2017/028182 A1, US 2004/078057 A1 and US 2010/121256 A1. These documents disclose all features in the preamble of claim 1.

### SUMMARY

The invention is defined by the claims.

### BRIEF DESCRIPTION OF THE DRAWINGS

Embodiments of the present invention are described below with reference to the attached drawings, in which:
FIG. 1A is a perspective view of an exemplary hearing prosthesis utilized in some exemplary embodiments;
FIG. 1B is a side view of the implantable components of the cochlear implant;
FIG. 2 is a side view of an embodiment of the electrode array illustrated in FIGs. 1A and 1B in a curled orientation;
FIG. 3 is a side view of an exemplary electrode array assembly;
FIG. 4 is a side view of the array of FIG 3 in a different state;
FIG. 5 is a side view of an alternate exemplary electrode array assembly;
FIGs. 6, 7, and 8 present a view looking down the longitudinal axis of some exemplary electrode array assemblies;
FIGs. 9-11 present side views of alternate embodiments of electrode array assemblies;
FIG. 12 presents a schematic that depicts an exemplary scenario of use according to an exemplary embodiment;
FIGs. 13-16 present further additional exemplary embodiments of electrode arrays;
FIG. 17 presents a view of an electrode array assembly inserted into a cochlea and inserted into a middle ear of the recipient;
FIG. 18 presents an exemplary alternate embodiment that is fundamentally different from all of the other embodiments disclosed herein;
FIGs. 19 and 20 depict alternate embodiments of the electrode array assembly that include a stylet passageway;
FIGs. 21 and 22 present yet alternate embodiments of electrode array assemblies;
FIGs. 23 and 24 present exemplary not claimed algorithms for exemplary methods; and
FIGs. 25 and 26 and 27 present alternate exemplary embodiments.

### DETAILED DESCRIPTION

It is noted that while various aspects of the present invention are described in the following with reference to a cochlear implant (whether it be a device utilizing electrodes or stimulating contacts that impart vibration and/or mechanical fluid movement within the cochlea), it will be understood that various aspects of the embodiments detailed herein are equally applicable to other stimulating medical devices having an array of electrical simulating electrodes such as auditory brain implant (ABI), functional electrical stimulation (FES), spinal cord stimulation (SCS), penetrating ABI electrodes (PABI), and so on. Further, it should be appreciated that the present invention is applicable to stimulating medical devices having electrical stimulating electrodes of all types such as straight electrodes, perimodiolar electrodes and short/basilar electrodes. Also, various aspects of the embodiments detailed herein and/or variations thereof are applicable to devices that are non-stimulating and/or have functionality different from stimulating tissue, such as for, example, any intra-body dynamic phenomenon (e.g., pressure, or other phenomenon consistent with the teachings detailed herein) measurement / sensing, etc., which can include use of these teachings to sense or otherwise detect a phenomenon at a location other than the cochlea (e.g., within a cavity containing the brain, the heart, etc.). Additionally, various aspects of the embodiments detailed herein and/or variations thereof are applicable to bone conduction devices, Direct Acoustic Cochlear Stimulators / Middle Ear Prostheses, and conventional acoustic hearing aids. Any device, system or method of evoking a hearing percept can be used in conjunction with the teachings detailed herein.
Accordingly, the claimed invention according to claim 1 addresses an apparatus that does not make reference to a specific cochlear implant. Instead, the claimed apparatus according to the features in the preamble of claim1 addresses more generally an apparatus that includes an implantable therapeutic substance reservoir and an implantable electrode array including a plurality of electrodes and a silicone carrier body, wherein the therapeutic substance reservoir is in fluid communication with the silicone carrier body.

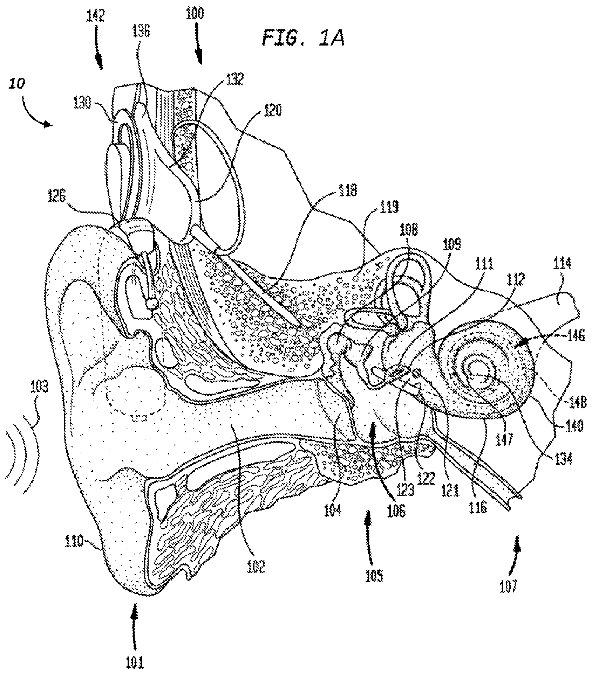

FIG. 1A is an illustrative example showing a perspective view of a totally implantable cochlear implant, referred to as cochlear implant 100, implanted in a recipient. The totally implantable cochlear implant 100 is part of a system 10 that can include external components, as will be detailed below.

The recipient has an outer ear 101, a middle ear 105 and an inner ear 107. Components of outer ear 101, middle ear 105, and inner ear 107 are described below, followed by a description of cochlear implant 100.

In a fully functional ear, outer ear 101 comprises an auricle 110 and an ear canal 102. An acoustic pressure or sound wave 103 is collected by auricle 110 and channeled into and through ear canal 102. Disposed across the distal end of ear canal 102 is a tympanic membrane 104 which vibrates in response to sound wave 103. This vibration is coupled to oval window or fenestra ovalis 112 through three bones of middle ear 105, collectively referred to as the ossicles 106 and comprising the malleus 108, the incus 109 and the stapes 111. Bones 108, 109 and 111 of middle ear 105 serve to filter and amplify sound wave 103, causing oval window 112 to articulate, or vibrate in response to vibration of tympanic membrane 104. This vibration sets up waves of fluid motion of the perilymph within cochlea 140. Such fluid motion, in turn, activates tiny hair cells (not shown) inside of cochlea 140. Activation of the hair cells causes appropriate nerve impulses to be generated and transferred through the spiral ganglion cells (not shown) and auditory nerve 114 to the brain (also not shown) where they are perceived as sound.

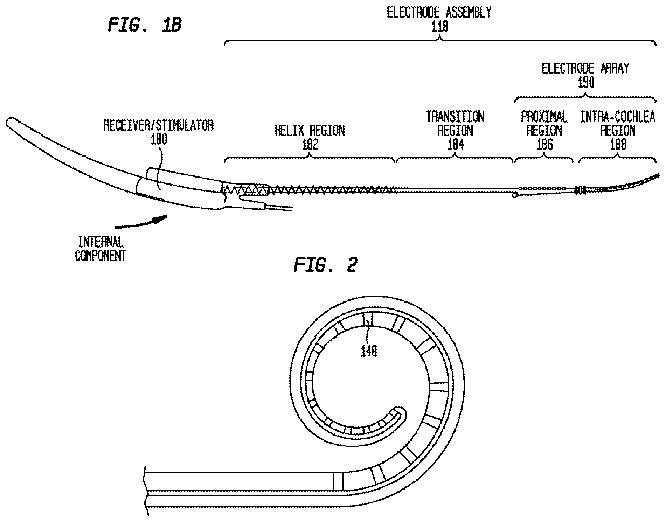

As shown, cochlear implant 100 comprises one or more components which are temporarily or permanently implanted in the recipient. Cochlear implant 100 is shown in FIG. 1 with an external device 142, that is part of system 10 (along with cochlear implant 100), which, as described below, is configured to provide power to the cochlear implant.

In the illustrative arrangement of FIG. 1A, external device 142 may comprise a power source (not shown) disposed in a Behind-The-Ear (BTE) unit 126. External device 142 also includes components of a transcutaneous energy transfer link, referred to as an external energy transfer assembly. The transcutaneous energy transfer link is used to transfer power and/or data to cochlear implant 100. Various types of energy transfer, such as infrared (IR), electromagnetic, capacitive and inductive transfer, may be used to transfer the power and/or data from external device 142 to cochlear implant 100. In the illustrative example of FIG. 1, the external energy transfer assembly comprises an external coil 130 that forms part of an inductive radio frequency (RF) communication link. External coil 130 is typically a wire antenna coil comprised of multiple turns of electrically insulated single-strand or multi-strand platinum or gold wire. External device 142 also includes a magnet (not shown) positioned within the turns of wire of external coil 130. It should be appreciated that the external device shown in FIG. 1 is merely illustrative, and other external devices may be used with embodiments of the present invention.

Cochlear implant 100 comprises an internal energy transfer assembly 132 which may be positioned in a recess of the temporal bone adjacent auricle 110 of the recipient. As detailed below, internal energy transfer assembly 132 is a component of the transcutaneous energy transfer link and receives power and/or data from external device 142. **In** the illustrative example, the energy transfer link comprises an inductive RF link, and internal energy transfer assembly 132 comprises a primary internal coil 136. Internal coil 136 is typically a wire antenna coil comprised of multiple turns of electrically insulated single-strand or multi-strand platinum or gold wire.

Cochlear implant 100 further comprises a main implantable component 120 and an elongate stimulating assembly 118. In embodiments of the present invention, internal energy transfer assembly 132 and main implantable component 120 are hermetically sealed within a biocompatible housing. In embodiments of the present invention, main implantable component 120 includes a sound processing unit (not shown) to convert the sound signals received by the implantable microphone in internal energy transfer assembly 132 to data signals. Main implantable component 120 further includes a stimulator unit (also not shown) which generates electrical stimulation signals based on the data signals. The electrical stimulation signals are delivered to the recipient via elongate stimulating assembly 118.

Elongate stimulating assembly 118 has a proximal end connected to main implantable component 120, and a distal end implanted in cochlea 140. Stimulating assembly 118 extends from main implantable component 120 to cochlea 140 through mastoid bone 119. In some embodiments stimulating assembly 118 may be implanted at least in basal region 116, and sometimes further. For example, stimulating assembly 118 may extend towards apical end of cochlea 140, referred to as cochlea apex 134. In certain circumstances, stimulating assembly 118 may be inserted into cochlea 140 via a cochleostomy 122. In other circumstances, a cochleostomy may be formed through round window 121, oval window 112, the promontory 123 or through an apical turn 147 of cochlea 140.

Stimulating assembly 118 comprises a longitudinally aligned and distally extending array 146 of electrodes 148, disposed along a length thereof. As noted, a stimulator unit generates stimulation signals which are applied by stimulating contacts 148, which, in an exemplary embodiment, are electrodes, to cochlea 140, thereby stimulating auditory nerve 114. In an exemplary embodiment, stimulation contacts can be any type of component that stimulates the cochlea (e.g., mechanical components, such as piezoelectric devices that move or vibrate, thus stimulating the cochlea (e.g., by inducing movement of the fluid in the cochlea), electrodes that apply current to the cochlea, etc.). Embodiments detailed herein will generally be described in terms of an electrode assembly 118 utilizing electrodes as elements 148. It is noted that alternate embodiments can utilize other types of stimulating devices. Any device, system or method of stimulating the cochlea via a device that is located in the cochlea can be utilized in at least some embodiments. In this regard, any implantable array that stimulates tissue, such as a retinal implant array, or a spinal array, or a pace maker array, etc., is encompassed within the teachings herein unless otherwise noted.

As noted, cochlear implant 100 comprises a totally implantable prosthesis that is capable of operating, at least for a period of time, without the need for external device 142. Therefore, cochlear implant 100 further comprises a rechargeable power source (not shown) that stores power received from external device 142. The power source may comprise, for example, a rechargeable battery. During operation of cochlear implant 100, the power stored by the power source is distributed to the various other implanted components as needed. The power source may be located in main implantable component 120, or disposed in a separate implanted location.

It is noted that the teachings detailed herein and/or variations thereof can be utilized with a non-totally implantable prosthesis. That is, in an alternate embodiment of the cochlear implant 100, the cochlear implant 100 is a traditional hearing prosthesis.

FIG. 1B is a side view of the internal component of cochlear implant 100 without the other components of system 10 (e.g., the external components). Cochlear implant 100 comprises a receiver/stimulator 180 (combination of main implantable component 120 and internal energy transfer assembly 132) and a stimulating assembly or lead 118. Stimulating assembly 118 includes a helix region 182, a transition region 184, a proximal region 186, and an intra-cochlear region 188. Proximal region 186 and intra-cochlear region 188 form an electrode array assembly 190. In an exemplary embodiment, proximal region 186 is located in the middle-ear cavity of the recipient after implantation of the intra-cochlear region 188 into the cochlea. Thus, proximal region 186 corresponds to a middle-ear cavity sub-section of the electrode array assembly 190. Electrode array assembly 190, and in particular, intra-cochlear region 188 of electrode array assembly 190, supports a plurality of electrode contacts 148. These electrode contacts 148 are each connected to a respective conductive pathway, such as wires, PCB traces, etc. (not shown) which are connected through lead 118 to receiver/stimulator 180, through which respective stimulating electrical signals for each electrode contact 148 travel.

FIG. 2 is a side view of electrode array assembly 190 in a curled orientation, as it would be when inserted in a recipient's cochlea, with electrode contacts 148 located on the inside of the curve. FIG. 2 depicts the electrode array of FIG. 1B in situ in a patient's cochlea 140.

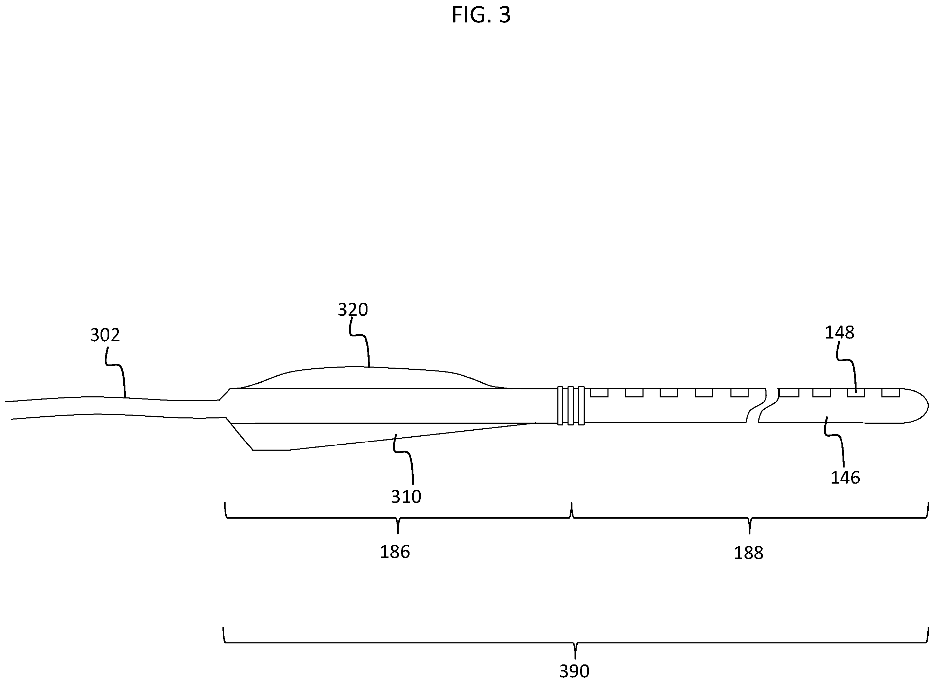

FIG. 3 depicts a side view of a device 390 corresponding to a cochlear implant electrode array assembly that can include some or all of the features of electrode array assembly 190 of FIG. 1B. More specifically, in an exemplary embodiment, stimulating assembly 118 includes electrode array assembly 390 instead of electrode array assembly 190 (i.e., 190 is replaced with 390).

Electrode array assembly 390 includes a cochlear implant electrode array componentry of the 190 assembly above. Note also element 310, which is a quasi-handle like device utilized with utilitarian value vis-à-vis inserting the 188 section into a cochlea. By way of example only and not by way of limitation, element 310, which is a silicone body that extends laterally away from the longitudinal axis of the electrode array assembly 390, and has a thickness that is less than that of the main body of the assembly (the portion through which the electrical leads that extend to the electrodes extend to the elongate lead assembly 302). The thickness combined with the material structure is sufficient so that the handle can be gripped at least by a tweezers or the like during implantation and by application of a force on to the tweezers, the force can be transferred into the electrode array assembly 390 so that section 188 can be inserted into the cochlea.

Also presented in figure 3 is reservoir 320. In an exemplary embodiment, reservoir 320 is configured to contain a bioactive substance or otherwise some form of mass that has fluid properties. In an exemplary embodiment, the reservoir 320 is in fluid communication with one or more portions of the electrode array making up section 188, as will be described in greater detail below. First however, some exemplary features of the reservoir will now be described.

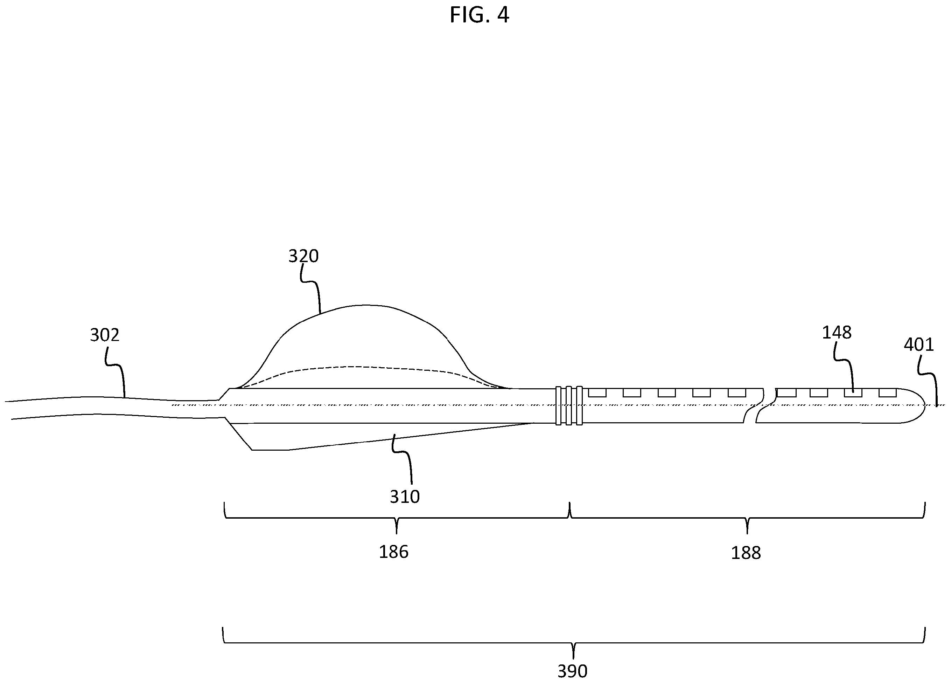

In an exemplary embodiment, the reservoir is an expandable reservoir. By way of example only and not by way of limitation, in an exemplary embodiment, the reservoir is made out of an elastomeric material and forms an elastomeric enclosure. In an exemplary embodiment, in a relaxed state, the reservoir 320 establishes a first interior volume and takes up a first exterior volume. When in an expanded state, the reservoir 320 establishes a second interior volume that is larger than the first interior volume, and also takes up a second exterior volume that is larger than the first exterior volume. FIG. 4 depicts an exemplary scenario of expansion of reservoir 320. In an exemplary embodiment, the second interior volume is less than, greater than or equal to 1.25, 1.5, 1.75, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19 or 20 or more times the first interior volume. In an exemplary embodiment, the second exterior volume is less than, greater than or equal to 1.25, 1.5, 1.75, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, 20, 21, 22, 23, 24, 25, 26, 27, 28, 29, 30, 31, 32, 33, 34, 35, 40, 45, 50, 55, 60, 65, 70, 75, 80, 85, 90, 100, 125, 150, 175 or 200 or more times the first exterior volume or any value or range of values therebetween on 0.01 increments). With respect to the exterior volume, this is the volume that is taken up by the reservoir itself and not the other components. That said, in an alternative embodiment, one can consider the entire electrode array assembly to establish a first overall exterior volume when the reservoir 320 is in the relaxed state, and a second overall exterior volume when the reservoir 320 is expanded. In an exemplary embodiment, the second overall exterior volume is less than, greater than or equal to 1.25, 1.5, 1.75, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, or 20 or more times the first overall exterior volume.

In an exemplary embodiment, the electrode array assembly 390 and/or the apparatus of which is a part, such as the implantable component of the cochlear implant, is configured to enable the reservoir to be filled after the electrode array assembly 390 is implanted in the recipient.

It is noted that the phrase "filled" as used herein is not an absolute term. This refers to the action of placing the substance into the reservoir from a location outside of the reservoir. The reservoir need not be filled to capacity.

In an exemplary embodiment, the aforementioned expansions can also occur after the electrode array has been inserted, including fully inserted, into the cochlea. In an exemplary embodiment, the filling of the reservoir can occur during the surgical operation and/or can occur after the surgical operation (as will be described in greater detail below). By during the surgical operation, it is meant while the opening accessing the middle ear cavity and the outside of the cochlea is open. This as distinguished from the temporal period after the opening is closed, which temporal period can encompass a period of time while the recipient is still in the operating room. Again, some of the features that enable the reservoir to be filled after the surgery will be described in greater detail below (and some of the features that enable the reservoir to be filled during the surgery will also be described).

In an exemplary embodiment, the electrode array assembly is configured such that the reservoir is located entirely in the middle ear cavity of the recipient after full operational expansion. By "full operational expansion," it is meant the expansion that results from the maximum expansion permitted by the supplier of the electrode array when the electrode array is fully implanted into the recipient. This as distinguished from full expansion, which encompasses the expansion just prior to structural failure of the reservoir. In an exemplary embodiment, the full operational expansion volumes can correspond to those detailed above and/or the full expansion can correspond to the volumes detailed above.

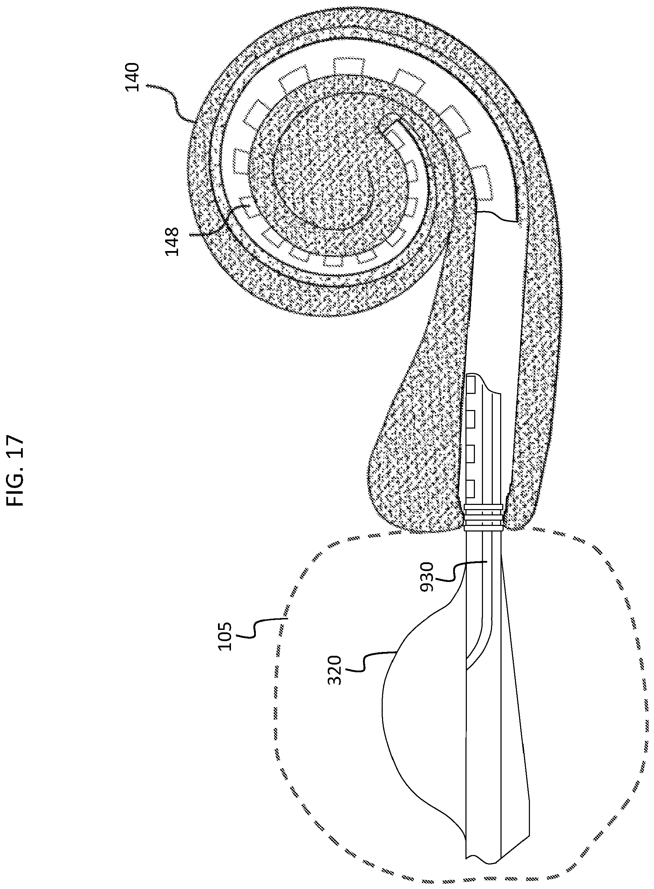

FIG. 17 presents a conceptual representation of the electrode array assembly 390 inserted into a cochlea 140 that is configured to prosthetically remain in the cochlea (that is, it is configured to remain in the cochlea for a time period concomitant with the use of a prosthetic device, as opposed to a temporary insertion such as might be the case for a needle or the like). FIG. 17 depicts a conceptual drawing depicting the intra-cochlea region 188 of the electrode array assembly 390 in the cochlea 140, and the proximal region 186 of the electrode array assembly 390 located outside the cochlea 140. Conduit 930 (more on this below) of the apparatus extends from inside the cochlea 140 to outside the cochlea into the middle ear cavity, which is functionally represented by the dashed enclosure 105, and to the reservoir 320.

It is noted that the concept in FIG. 17 is just that - conceptual, and is provided at least for the purpose of presenting the concept of the cochlear implant electrode array having the assembly 390 only partially inserted into the cochlea (but this represents full insertion of the electrode array). In an exemplary embodiment, the electrode array assembly along with a portion of the conduit 930 is inserted into the scala tympani. Accordingly, in an exemplary embodiment, there is an electrode array assembly configured such that the electrode array is insertable into the scala tympani, and the reservoir is located in the middle ear cavity outside the scala vestibule but in fluid communication at least indirectly therewith.

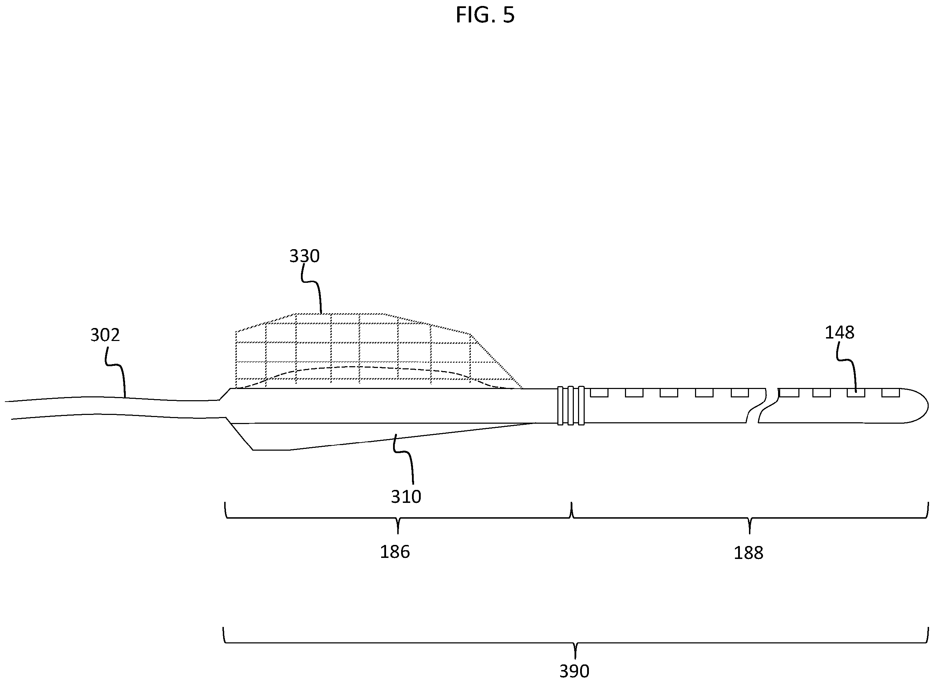

Briefly, it is noted that in an exemplary embodiment, a protective apparatus can be placed around the expandable reservoir. This is seen by way of example only and not by way of limitation, in figure 5, where cage 330 surrounds the reservoir 320. In an exemplary embodiment, cage 330 prevents further expansion of the reservoir 320. In an exemplary embodiment, this can prevent overexpansion of the reservoir, and thus reduce the statistical chances that the reservoir will burst during filling relative to that which would be the case in the absence of cage 330. In an exemplary embodiment, element 330 is not a cage but instead a walled structure of solid walls with few if any passageways therethrough (save for a pressure relief orifice or the like that is present so that the fluid between the outer surface of the reservoir and the inner surface of the walled structure can exit the interior of the wall structure during "inflation" of the reservoir and can enter the interior volume during "deflation" of the reservoir, etc. In an exemplary embodiment, cage 330 is collapsible. In an exemplary embodiment, the cage 330 can be more of a net or the like. The cage 330 can be bunched up like a net, and then upon the expansion of the reservoir 320, the cage will unbunch, but only to a certain extent. Alternatively, and/or in addition to this, the cage can be configured to be bunched under a force, but when that force is relieved, the cage will spring back to its original form. This can provide a protection regime for the reservoir while also permitting the cage to be "moved" in the event that such is utilitarian with respect to implanting the electrode array assembly into a recipient.

In an exemplary embodiment, the reservoir can be a sponge or the like. In an exemplary embodiment, the sponge can be enclosed in a non-permeable membrane. The sponge can be configured to expand upon "soaking up" therapeutic substance or the like, and then be of a configuration that the sponge shrinks over time, thus resulting in the transfer of the therapeutic substance into the conduit. In an exemplary embodiment, the sponge can be a substance that contracts when exposed to heat, such as body heat, but by providing a relatively cold therapeutic substance, the sponge will expand, thus soaking up the therapeutic substance. Note also that in an exemplary embodiment, the net or the like where the membrane that is configured to be elastically expanded can be utilized so as to put pressure on the sponge to "squeeze" the therapeutic substance there from.

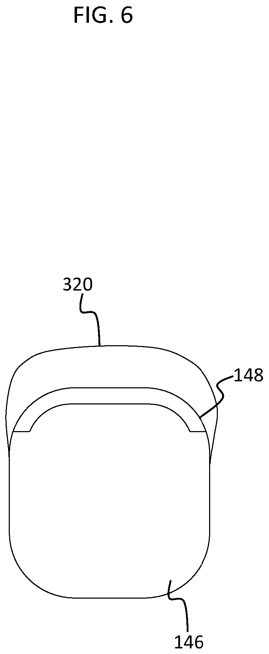

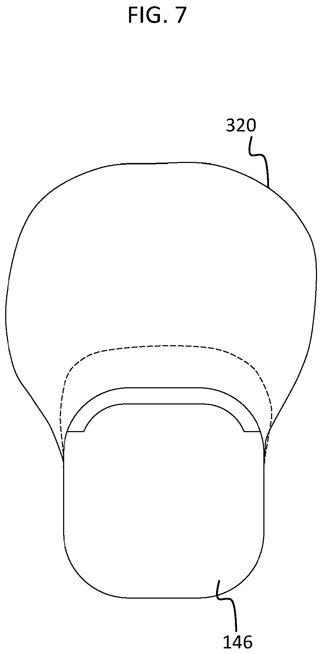

FIGs. 6 and 7 pictorially represent views of the electrode array assembly looking down the longitudinal axis and respectively represents the reservoir 320 in the relaxed state and in the expanded state. It is noted that while the embodiments depicted in figures 5-7 present the reservoir located on the "top" (relative to the views of these figures) of the electrode array assembly, in alternative embodiments, the reservoir can be located on the side either side and/or on the bottom. Moreover, while the embodiments depicted in figures 6 and 7 depict the reservoir expanding upwards (again, relative to the figure) and slightly outwards, in other embodiments, the reservoir can expand sideways instead of upwards and/or both sideways and upwards. Indeed, in an exemplary embodiment, the cage can be structured to "guide" the expansion of the reservoir in directions that is deemed utilitarian. By way of example only and not by way of limitation, the cage can extend about the longitudinal axis of the electrode array assembly, and during expansion, the reservoir might extend upwards until it hits the cage, and then expand outwards and then be guided downwards around either side of the longitudinal axis of the electrode array assembly, and then even possibly beneath the longitudinal axis and/or then possibly inwards. Any arrangement of expansion that can have utilitarian value can be utilized in at least some exemplary embodiments.

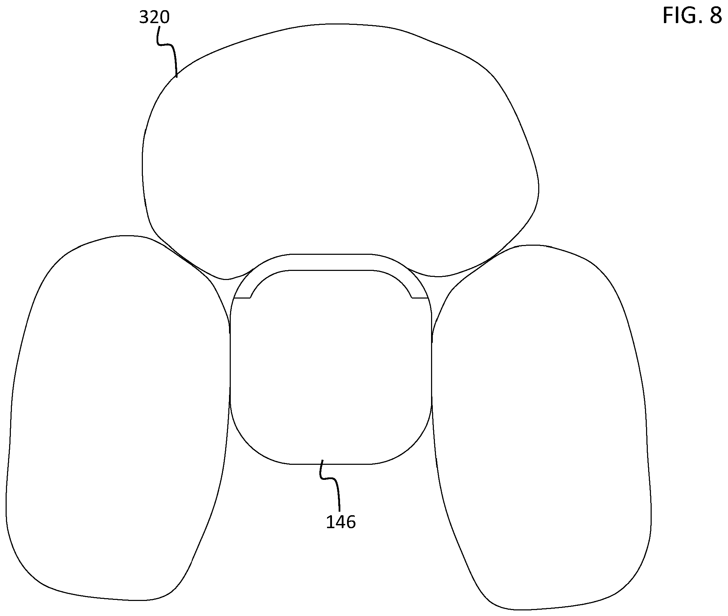

Note also that while the embodiments depicted above have focused on the utilization of a single reservoir, in an alternate embodiment, two or more reservoirs can be utilized. Figure 8 depicts an exemplary embodiment where three separate reservoirs are utilized. The reservoirs can be manifold to one another so that the reservoirs expand and/or contract at a similar (which includes the same) rate as each other, or can be fluidically separated from each other so that expansion and/or contraction of one as a result of mass flow in and/or out of that one reservoir does not affect the pressure in the other reservoirs. In an exemplary embodiment, two, three, four, five, six, seven, eight, nine, and/or ten or more separate reservoirs can be present, all of which would be located, at least in some exemplary embodiments, in the middle ear cavity upon full implantation of the electrode array assembly in the recipient, before and/or after full expansion of all of those reservoirs. It is noted that in some embodiments, the reservoir or one or all of the reservoirs or a portion of one or more can be located at other locations other than the middle ear. Indeed, as will be detailed below, the reservoir or a portion thereof or more than one or a portion of more than one can be located in whole or in part in the cochlea. In some embodiments, all or part of the reservoir is located in a surgical cavity created during surgery.

The utilization of two or more reservoirs can have utilitarian value with respect to providing a "spare" reservoir. If one reservoir ultimately becomes less than fully functional, the other reservoir can be utilized in its place. Alternatively, and/or in addition to this, one or more reservoir can be utilized to deliver drug into the cochlea while one or more other reservoirs are being refilled. More on this below. Briefly, it is noted that two or more drugs / therapeutic substances can be delivered. Thus, one therapeutic substance can be delivered while refilling of another, different therapeutic substance, etc.

It is briefly noted that frequently, the phrase "drug" will be utilized herein. Embodiments are directed towards a drug delivery system. However, embodiments are not so limited unless otherwise specified. In this regard, embodiments are directed towards a therapeutic substance delivery system. Therapeutic substances include drugs, but also include nondrug substances. In an exemplary embodiment, therapeutic substances include steroids and biologics. Therapeutic substances can also include minerals and the like. Any disclosure herein of drug or the containment of drug or the delivery of drug also corresponds to another embodiment that corresponds to an embodiment that is directed towards a therapeutic substance. That is, typically, the word drug used herein is shorthand for therapeutic substance. Accordingly, embodiments include the present disclosure where the word drug is replaced by the word therapeutic substance, unless otherwise specified.

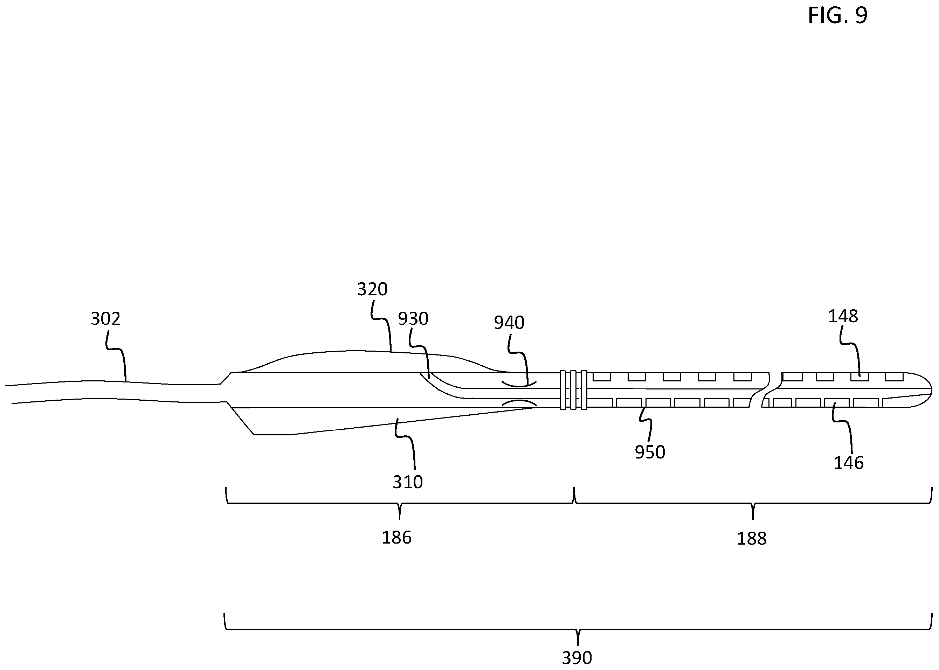

Figure 9 depicts a cross-sectional view of the electrode array assembly 390 with the reservoir 320 in the relaxed state. As can be seen, conduit 930 extends from the reservoir into section 188, and then along the length of section 188. Also as can be seen, sub conduits extend radially away from the longitudinal axis of the conduit 930, and lead to orifices 950, the system enabling mass flow from the reservoir 320 into the cochlea. As can be seen in this exemplary embodiment, an optional flow restrictor 940 is located in section 186, between the reservoir 320 and the intracochlear portion / the orifices that lead into the cochlea. Alternatively, and/or in addition to this, flow restrictor 940 can be located in section 188. It is also noted that in at least some exemplary embodiments, there is no flow restrictor 940 (more on this below). The utility of the flow restrictor will be described in greater detail below. In an exemplary embodiment, the flow restrictor is a membrane or the like, such as a restrictive membrane, that enable the controlled release of the therapeutic substance.

In an exemplary embodiment, a filter is utilized. In some embodiments, the filter serves a dual function as a flow restrictor, while in other embodiments, the filter is a standalone filter. In an exemplary embodiment, a 22 µm filter is utilized, which is configured to exclude bacteria and/or restrict fluid flow therethrough. In an exemplary embodiment, the filter thus is configured to exclude bacteria. Filters of the larger filter size and/or smaller filter size can be utilized in at least some exemplary embodiments.

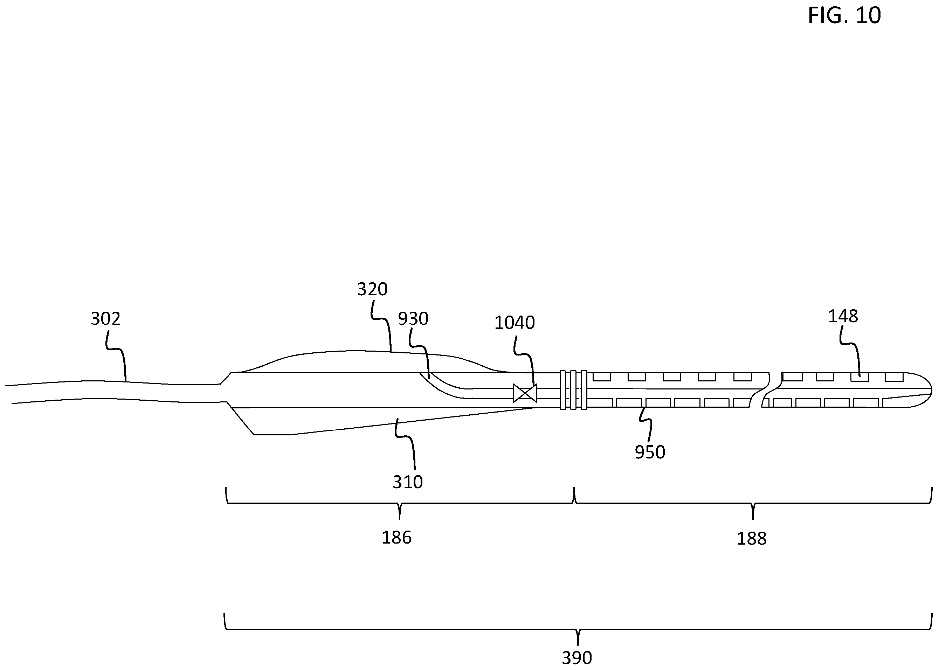

Figure 10 presents an alternate embodiment where valve 1040 is located between reservoir 320 and the orifices 950. In an exemplary embodiment, valve 1040 has utility with respect to enabling the metering or otherwise enabling the control of the mass flow from the reservoir 320 and/or into the cochlea. By way of example only and not by way of limitation, valve 1040 can be a valve that is includes an electrical solenoid or the like, which solenoid is operated or otherwise controlled by an electrical signal. The electrical signal can be supplied from the receiver stimulator, by way of example only and not by way of limitation. In this regard, in an exemplary embodiment, the implantable component and/or the external component can be programmed so as to provide the control signal so as to open and/or close the valve. Moreover, in an exemplary embodiment, the receiver stimulator and/or the external component can be programmed or otherwise can be loaded with a drug delivery regime data set that will open and/or close the valve based on a temporal schedule and/or based on some other action/reaction paradigm that can be developed to have utilitarian value. Some additional utilitarian features of the valve will be described in greater detail below.

In view of the above, as can be seen, in an exemplary embodiment, there is an apparatus, comprising, by way of example, a cochlear implant electrode array, and an implantable drug reservoir. In this exemplary embodiment, the apparatus is configured such that the drug reservoir is in fluid communication with the electrode array, and the drug reservoir is at least substantially located in a middle ear space when the cochlear implant electrode array is fully implanted in a recipient. In still further embodiments, the drug reservoir is totally located in a middle ear space when the cochlear implant electrode array is fully implanted in a recipient, both in the relaxed state and in the fully operational expanded state. In this regard, in at least some exemplary embodiments, the reservoir is an elastomeric enclosure that expands upon the insertion of a liquid drug therein. Further, in at least some exemplary embodiments, the expansion maintains the liquid drug under pressure, and forces the drug out of the reservoir and into the electrode array over time. As will be described in greater detail below, there are utilitarian rates of drug flow into the cochlea. In an exemplary embodiment, such devices as the flow restrictor 940 detailed above permits the fluid to only flow slowly out of the reservoir. In an exemplary embodiment, the valve 1040 can permit the fluid to flow at a fast rate in a temporally localized manner, but at a slow rate in a temporally globally manner (e.g., the rate may be fast for a second, but because it only occurs for one second every hour or every day, etc., the global rate is slow).

Note also that in at least some exemplary embodiments, the material of the reservoir is such that the material contracts slowly, even in the absence of internal pressure that established, for example, the maximum expansion. By way of example only and not by way of limitation, this could be the antithesis of a party balloon, where, for example, the air pressure inside the party balloon is what maintains the party balloon in the expanded state. In this exemplary embodiment now being described, the reservoir can be slow to contract even when the pressure inside the reservoir drops below that which cause the reservoir to expand to the volume at that time. Thus, in an exemplary embodiment, irrespective of whether or not there exists a flow restrictor or a valve, it is the contraction of the reservoir itself that can cause the drug to flow slowly into the cochlea, because the reservoir itself is taking its time to contract, and as the reservoir contracts, it forces more and more drug out of the electrode array assembly and into the cochlea. Still, in at least some exemplary embodiments, it is the valves and the flow restrictors, and other componentry and other features of the electrode array assembly as described below that control the rate of flow into the cochlea, and thus control the expansion of the reservoir.

Thus, in an exemplary embodiment, the apparatus includes a valve, wherein the valve controls flow of the drug from the reservoir into the electrode array. That said, in an alternate embodiment, the apparatus is valve-less with respect to fluid communication between the reservoir and the inside of the cochlea. Alternatively, or in addition to the utilization of a valve, the apparatus includes a flow restrictor that restricts flow out of the reservoir to the electrode array beyond that which would otherwise be the case in the absence of the flow restrictor. When the phrase "flow restrictor" is used herein, it is meant an affirmative structure that is in addition to the structure of the electrode array assembly which would be present without the drug delivery system. In this regard, as will be described in greater detail below, in some exemplary embodiments, the carrier member of the electrode array is utilized to quasi-meter the flow of drug into the cochlea from the reservoir. This carrier member would be present irrespective of the drug delivery system, and thus does not constitute a flow restrictor as that phrase is used herein. Indeed, in at least some exemplary embodiments, the utilization of the carrier member enables a valve-less and a flow restrictor-less apparatus. Some additional details of this will be described in greater detail below.

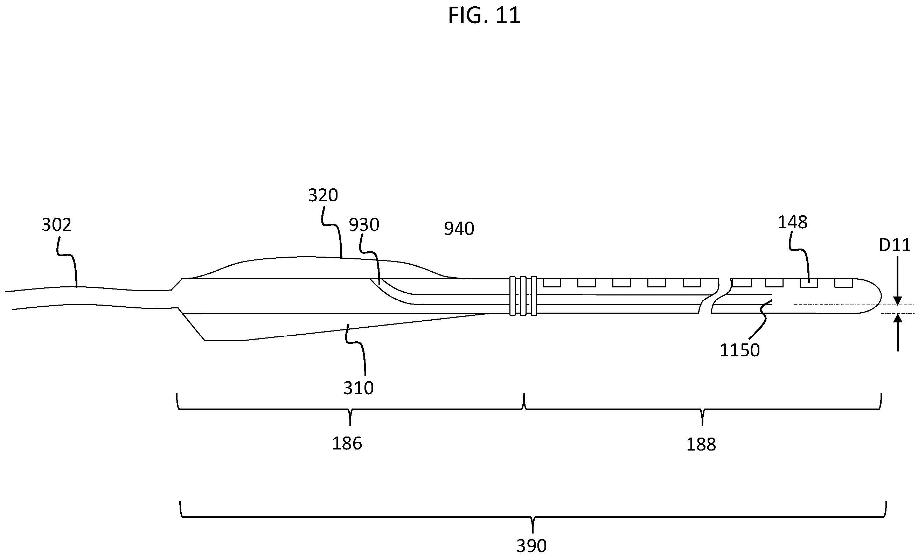

In some embodiments of this embodiment, the electrode array is configured such that the drug elutes from the array upon entering the array and passes into the cochlea via the drug passing from the conduit 940 out the end of the conduit 1150 and into the carrier member, which is made of silicone, and, in some embodiments, saturates at least a local portion of the carrier member, whereby the drug then elutes from the carrier member, or more accurately, from that local portion of the carrier member, into the cochlea. Figure 11 depicts an exemplary embodiment, which does not include the valve and/or the flow restrictor (although some other embodiments can include the valve and/or the flow restrictor - note embodiments can include both a valve and a flow restrictor), where the end of the conduit 1150 would be considered "sealed" by the carrier member body (the silicone of the carrier member body), as the silicone extends from one side of the end of the conduit to the other side of the end of the conduit. Indeed, in an exemplary embodiment, where the conduit is established by, for example, something more than simply a hollow within the silicone body that establishes the carrier member, such as for example a tube and/or a micro-tube, which can be made out of a biocompatible material or the like and/or made out of a material that is compatible with the substance to be transferred into the cochlea, a portion of the carrier member can actually extend within the tube, and can be a plug like component. That said, in an alternative embodiment, there can be a cavity within the carrier member at the end of the tube that establishes the conduit, where the drug or the like can pool or otherwise accumulate. Any arrangement that can have utilitarian value that can enable the teachings detailed herein can be utilized in at least some exemplary embodiments.

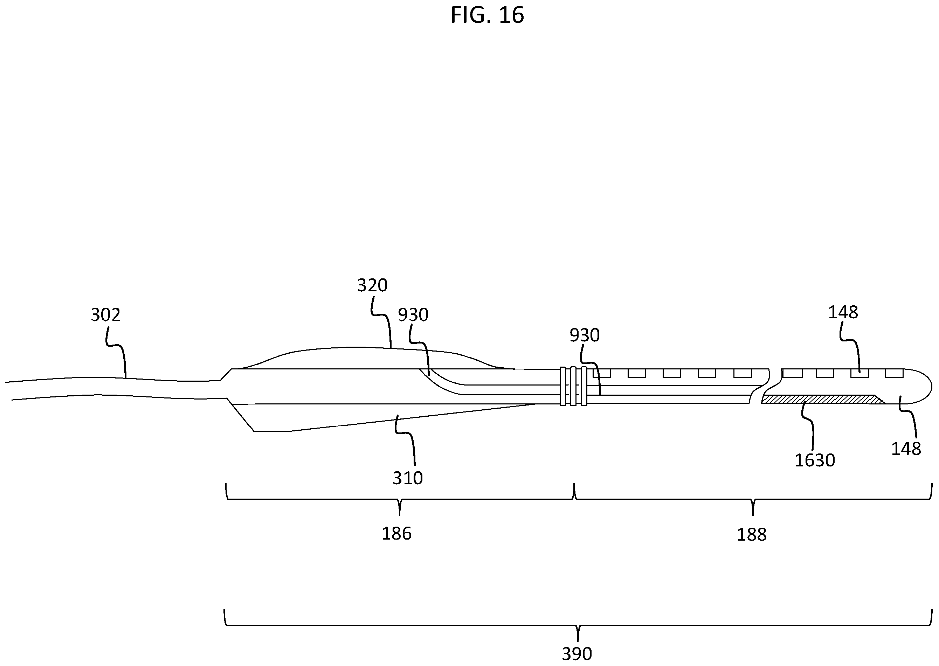

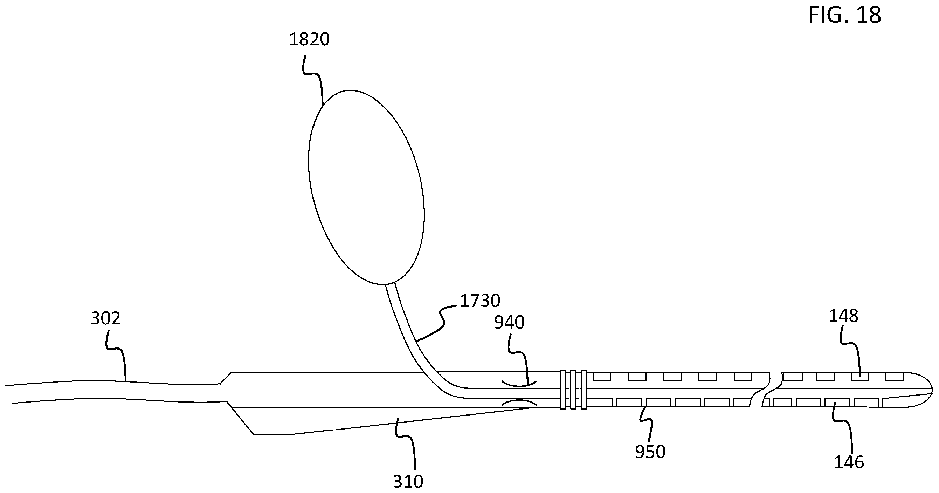

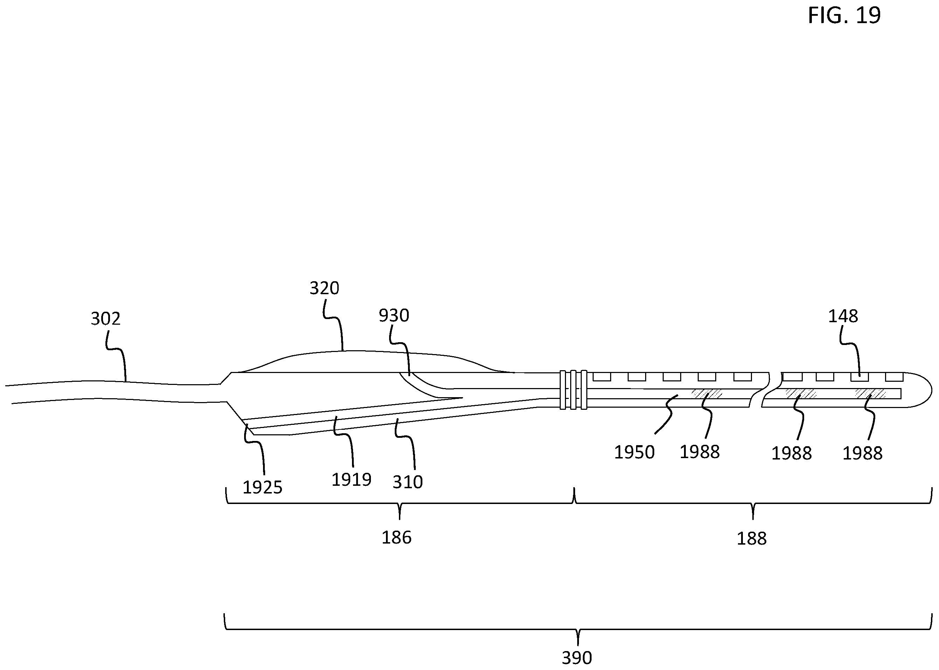

Figure 11 includes dimension D11. In an exemplary embodiment, the distance of the passageway 930 from the outer surface of the electrode array can impact the delivery rate and/or the amount of substance that is delivered. That is, in an exemplary embodiment, designs of the electrode array can be devised that regulate the delivery rate as a function of proximity of the delivery passage to an outer surface of the array. By way of example only, when the passageway/channel is closer to the longitudinal axis of the array, the drug must pass through more material to escape or otherwise leave the array, and vice versa, all other things being equal (e.g., same diameter of the passageway, etc.). Conversely, when the passageway/channel is closer to the outer surface of the array, the drug must pass through less material to escape or otherwise leave the array, and vice versa. Thus, in an exemplary embodiment, the distance D11 from the surface of the passageway to the outer surface of the electrode array can control the rate of delivery. This can be the case with respect to the embodiment shown in figure 11, or any other of the embodiments where the therapeutic substance diffuses along the length of the passageway. Accordingly, in some exemplary embodiments, there are methods of designing the electrode array so that the rate of diffusion or the rate of delivery of the therapeutic substance is less than that which would be the case if the passage was closer to the surface and/or further from the longitudinal axis of the array, and vice versa. This principle can be the case with respect to other embodiments detailed herein. Indeed, jumping ahead, this principle can be the case with respect to element 1630 of figure 16 as will be described below, by way of example and not by way of limitation. This principle can also be the case with respect to, for example, the embodiment of figure 18, where passageways 950 are filled with a substance that provides resistance to flow from the passageway to the surface of the array (e.g., aerated silicone). The greater the distance of the passageways that are filled with this resistance substance, the lower the rate of diffusion. This is also the case with respect to the embodiment of figure 19 again by example and not by limitation.

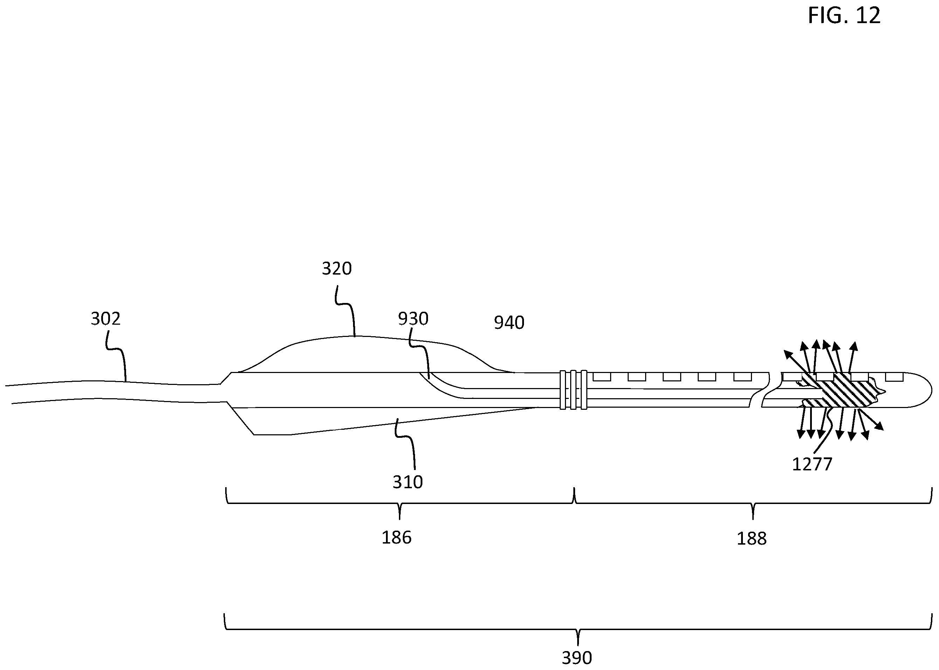

Figure 12 provides an exemplary embodiment where a portion of the carrier member is saturated with drug (portion 1277) owing to the drug flowing through the conduit 930 under pressure from the reservoir 320 (which is shown in a contracted state relative to its expanded state). Drug delivery is represented by the arrows emanating out of portion 1277. It is noted that the arrows are only present at the surface where the carrier member is saturated by the drug. That is, in at least some exemplary embodiments, the drug does not elute or otherwise does not effectively elute from surfaces where the adjacent body of the carrier member is not saturated. In an exemplary embodiment, the amount of drug that leaves the surfaces where the underlying body is not saturated vs. the surfaces where the underlying body is saturated is less than 50, 60, 70, 75, 80, 85, 86, 87, 88, 89, 90, 91, 92, 93, 94, 95, 96, 97, 98, 99, or 100% of that which leads the latter. It is noted that the boundaries of saturation with distance from the end of the conduit can vary depending on the pressure and/or the drug and/or the structure of the carrier member, etc.

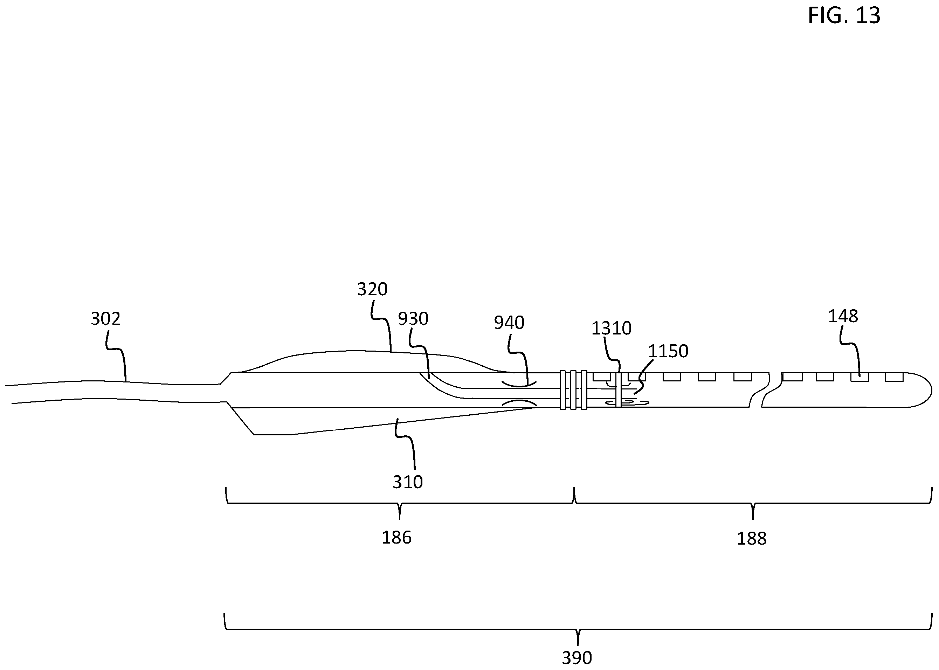

Figure 13 depicts an alternate embodiment of the electrode array assembly 390, where the end of the conduit 1150 and that a location that is closer to the boundary between section 186, the extra-cochlear section, and section 188, the intra-cochlear section. As can be seen, in this exemplary embodiment, a barrier 1310 is provided between the beginning of section 188 and end of the conduit, which in this embodiment, also includes a tube structure establishing the conduit, which extends through the barrier 1310. In an exemplary embodiment, the barrier 1310 is configured to prevent or otherwise limit the amount of drug that can move from the location, with respect to the figure, on the right of barrier 1310, to a location on the left of barrier 1310. In an exemplary embodiment, this can have utilitarian value with respect to preventing and/or otherwise limiting the amount of drug that moves from section 188 into section 186, at least in a scenario where, for example, the material of the carrier member is saturated with the drug.

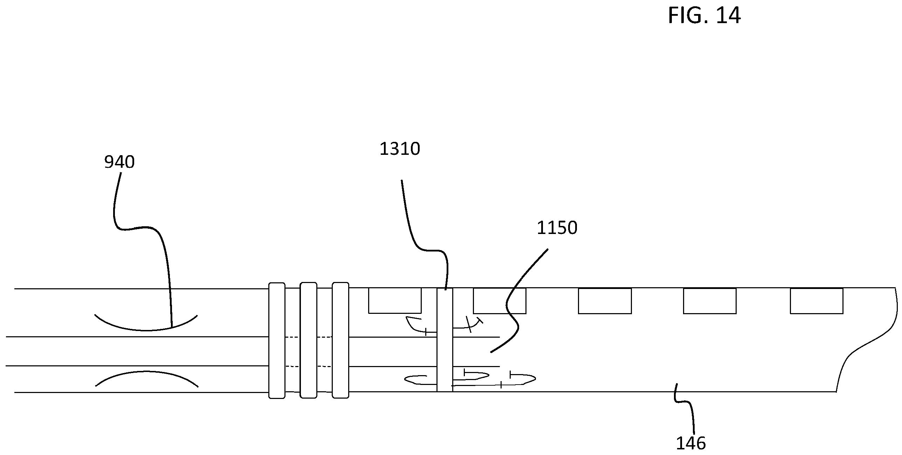

FIG. 14 provides a close-up view of the barrier 1310. Here, the barrier 1310 extends substantially if not all the way across the carrier member 146. In some embodiments, it completely bisects the material of the carrier member. In other embodiments, portions of the carrier member can extend over / around and/or through the barrier 1310. In the embodiment shown, hooks 1444 are shown extending from the faces of the barrier 1310. In an exemplary embodiment, when the carrier member is molded during the molding operation to establish the carrier member, the carrier member is also molded around these hooks, and thus the hooks hold the two sides of the carrier member together whereas otherwise the only thing that would hold the two sides would be the tube and/or the electrical leads to the electrodes. In an exemplary embodiment, the hooks can extend further in the longitudinal direction than that shown.

In an exemplary embodiment, the barrier can be considered a porous structure that will permit the silicone to flow into the structure and thus "grip" the cured silicone to hold the two bodies together. While in some scenarios, drug may be able to pass through the porous body from one side to the other, but the amount that would occur would be limited relative to that which would be the case in the absence of the barrier. Alternatively, and/or in addition to a porous barrier, a barrier with discrete holes can be utilized, where the material of the silicone flows through during the molding operation, and thus there are silicone fingers that extend from one side of the barrier to the other side of the barrier that hold the body together. Any device, system, and/or method that can hold two separate bodies of silicone together that can enable the teachings detailed herein so as to at least approximate, in performance function, that structure which would result in the absence of the barrier 1310, can be utilized in at least some exemplary embodiments.

In an exemplary embodiment, barrier 1310 is a titanium disk through which a hole extends for the tube of the conduit and/or for leads. In an exemplary embodiment, the disk could be another material, such as, for example PEEK. Any material or arrangement that can enable the utility of the titanium desk can be utilized at least some exemplary embodiments. In another exemplary embodiment, filled silicone can be used at a level, such as a heavily filled silicone, so that such allows little passage of the drug therethrough.

It is noted that the embodiment of figures 13 and 14 include the flow restrictor 940, even though the carrier member 146 utilized as the elutor and itself can limit the flow of the drug out of the reservoir. Still, in alternate embodiments of this embodiment, the flow restrictor 940 is not present (the valve can be present, or is not present as well).

In an exemplary embodiment, the drug reservoir is a separate component of the electrode array assembly relative to the other components of the electrode array assembly, and is mechanically and/or adhesively connected, etc., to one or more or other components of the electrode array assembly. That said, in an alternate embodiment, the drug reservoir is an integral part of at least some of the other components of the electrode array assembly. By way of example only and not by way of limitation, the elastomeric material can be an extension of the base material of the section 186. By way of example only and not by way of limitation, while the carrier body 146 of section 188 can be made of silicone, a transition region can be present, such as at a location in section 186, where the main structure of the electrode array assembly transitions to another material that is the same as the material that constitutes the elastomeric reservoir. That said, in an exemplary embodiment, the elastomeric reservoir can also be made of a silicone, albeit perhaps a different grade and/or a different type of silicone then that which makes up the carrier 146 of section 188, such as one that is more stretchable relative to the material in section 188. Alternatively, and/or in addition to this, the silicone of the reservoir can be a composite structure or the like, where the elastomeric material is embedded in the silicone or otherwise located with the silicone. Still further, a material that retains the substance to be located in the reservoir can be combined with the elastomeric material and/or the silicone structure of the reservoir. In this regard, this material that retains the substance to be located in the reservoir, which could be, for example, a foil that readily collapses and on collapsation, does not provide the elastomeric features of the reservoir, but instead provides the drug reservoir features only. In an embodiment, the material can function as a liner or the like, and the elastomeric features can be achieved by the component that is lined by the liner.

FIG. 18 depicts an alternate embodiment in which the reservoir is a separate unit from the electrode array assembly. More specifically, FIG. 18 depicts an assembly 1890 which includes an electrode array and a reservoir, reservoir 1820, which is an elastomeric reservoir in this embodiment. However, reservoir 1820 is a remote separate unit from the electrode array assembly, albeit in fluid and pressure communication with the electrode array assembly via conduit 1830. Thus, in contrasting the embodiment of, for example, FIGs. 4 and 8, from the embodiment of FIG. 18, it can be seen that in the embodiment of FIGs. 4 and 18, the reservoir and the electrode array are a single unit (i.e., the electrode array assembly 390 is a combined electrode array and the reservoir(s)). This is as differentiated from, for example, an electrode array assembly 190 to which, by way of example, one or more or all the components of the reservoir are merely attached or otherwise linked to in a non-unitized manner, such as is the case with respect to the embodiment of FIG. 18, where the drug delivery apparatus includes portions which are part of the unit of the electrode array assembly and portions which are not part of the unit of the electrode array assembly (e.g., the reservoir 120). In the exemplary embodiment of some of the embodiments of FIGs. 4 and 8, the structure of the electrode array assembly 390 is such that the drug delivery system in general, and the reservoir in particular is an integral part of the electrode array assembly 390, just as is the case with the electrode array. Note that the reservoir is distinct from the delivery passage through the array, as is the case in the embodiments of FIGs. 4 and 8 and 18

According to the claimed invention, the passageway through the array is formed by a porous and/or an aerated silicone that enables the drug to pass between the voids. In an exemplary embodiment, this functions as a flow restrictor and/or regulator. In an exemplary embodiment, the body of the electrode array is formed of two separate silicone bodies. In an exemplary embodiment, the passageway is a first body that is previously formed (e.g., it can be a tube or it can be a porous / aerated silicone body as noted above) and the remainder of the silicone body is formed around this first body, thus establishing the carrier of the electrode array. Still further, in an exemplary embodiment, the passageway can be formed contemporaneously with the rest of the body, such as, for example, by controllably aerating just a portion of the silicone. Still further, in an exemplary embodiment, the first body of aerated or porous silicone can be encapsulated in a tube or the like or some other barrier, which separates the aerated silicone from the rest of the silicone. This tube can have openings as needed or otherwise as utilitarian to enable the therapeutic substance to flow from the first body. In this regard, the aerated silicone functions more as a flow restrictor or the like as opposed to a passageway. Note also that in an exemplary embodiment, the outlets can be established by the aerated/porous silicone, which can serve as a flow restrictor as well.

In an exemplary embodiment, the porosity and/or the geometry of the aerated/porous silicone is controlled so as to achieve a given flow rate or otherwise a desired flow rate with respect to a given back pressure, etc., or irrespective of a given back pressure. In this manner, in at least some exemplary embodiments, such can achieve the flow restrictions / regulation features disclosed herein and/or variations thereof.

It is further noted that in at least some exemplary embodiments, the electrode array assembly 390 is configured such that the reservoir 320 is fixed relative to position along the longitudinal axis 401, where the longitudinal axis extends through the electrode array. This embodiment is in contrast to the embodiment of FIG. 18, where the conduit 1830 enables the reservoir 1720 to move relative to position along the longitudinal axis of the electrode array assembly.

It is also noted that with respect to the embodiments of the cage detailed above, the cage itself could be an elastomeric structure. In this regard, in an exemplary embodiment, a collapsed bag or the like can be located inside the cage structure. The cage structure itself can also be collapsed. As the collapsed bag is filled with fluid, such as a drug or other beneficial substance, the bag uncollapses, and the cage expands outward, in an elastomeric manner, and the dimensions of the cage, or more accurately, the passageways through the cage (the grid) are sized and dimensioned to provide pressure against the bag uncollapsing. As the bag uncollapses, it puts outward pressure under the cage, which is resisted in part by the cage, thus creating pressure inside the bag.

Any arrangement that can enable the teachings detailed herein can be utilized in at least some exemplary embodiments.

It is noted that in at least some exemplary embodiments, the apparatus is pump-less. In an exemplary embodiment, the pressurized delivery of the drug into the cochlea is accomplished without a pump. In an exemplary embodiment, drug delivery occurs, at least for substantially all of the time that drug delivery is occurring, without the utilization of any electricity and/or electromagnetic systems, other than that which may be utilized to start and/or stop the drug delivery. In an exemplary embodiment, drug delivery occurs, at least for 80, 81, 82 83, 84, 85, 86, 87, 88, 89, 90, 91, 92, 93, 94, 95, 96, 97, 98, 99, or 100% of the time that drug delivery is occurring, without the utilization of any electricity and/or electromagnetic systems, other than that which may be utilized to start and/or stop the drug delivery. It is noted that the aforementioned temporal periods, in at least some exemplary embodiments, are contiguous uninterrupted periods. That is, by way of example only and not by way of limitation, in an exemplary embodiment, if the percentage is at least 99 percent, this means that out of 100 hours, there is a period of at least 99 hours of drug delivery uninterrupted by the utilization of electricity and/or electromagnetic systems, other than to start and/or stop the drug delivery. That said, in other embodiments, the aforementioned temporal periods are not so uninterrupted.

In at least some exemplary embodiments, the apparatus is configured to enable refilling of the reservoir without explanting the reservoir and/or without removing the reservoir from inside the recipient. Indeed, in an exemplary embodiment, the apparatus is configured to enable refilling of the reservoir without any additional procedures beyond that which resulted in the reservoir being implanted in the recipient and the first instance. In an exemplary embodiment, the apparatus is configured to enable refilling of the reservoir utilizing completely supercutaneous devices, which devices never extend below the skin of the recipient into the recipient. That said, in an exemplary embodiment, the apparatus is configured to enable refilling of the reservoir utilizing percutaneous devices that can enter the skin and into the recipient via a passage that has a maximum diameter no greater than 5, 4, 3.5, 3, 2.5, 2, 1.5, 1.4, 1.3, 1.2, 1.1, 1.0, 0.09, 0.8, 0.7, 0.6, 0.5, 0.4, 0.3, or 0.2 mm. Additional details of this feature will be described in greater detail below.

In view of the above, it can be seen that in an exemplary embodiment, there is an implantable apparatus, comprising an implantable drug reservoir, such as by way of example only and not by way of limitation, drug reservoir 320, and an implantable electrode array, the array including a plurality of electrodes and a silicone carrier body. In this exemplary embodiment, the drug reservoir is in fluid communication with the silicone carrier body and the silicone body at least one of restricts the flow of drug out of the drug reservoir or enables the drug to elute through the silicone carrier body. With respect to the following embodiments, the embodiments will be described respect to the silicone body enabling the drug to elute through the silicone carrier body.

In an exemplary embodiment, the aforementioned apparatus is a cochlear implant electrode array. That said, it is to be understood that in at least some exemplary alternate embodiments, the array is a different type of array, such as by way of example only and not by way of limitation, an array of a retinal implant, a vestibular implant, etc.

In at least some exemplary embodiments, the apparatus is configured such that the reservoir at least one of periodically loads the carrier body with drug or maintains the carrier body loaded with drug. By loaded, it is meant that the carrier body is in a state where the drug elutes from the carrier body. The embodiment of figure 12 would be considered a loaded carrier body, even though portions of the carrier body are not saturated or otherwise do not contain sufficient amounts of the drug to elute from the surfaces established by the body.

By utilizing a valve, which valve is controlled to be turned on and turned off, the carrier body can be periodically loaded with the drug. After an amount of the drug has eluted out of the carrier body, and thus the carrier body is no longer loaded, the valve can be opened, and more drug can flow into the carrier body, and thus the carrier body can be loaded again. Alternatively, the carrier body is loaded continuously by maintaining the valve open or otherwise dispensing with a valve.

It is also noted that the embodiment of figure 14 can be utilized downstream of the opening 1150 of the conduit. In this regard, in an exemplary embodiment, a control volume of the carrier body that can be saturated can be established. In this regard, the barrier 1310 that is downstream from the opening establishes a barrier that prevents the therapeutic substance from traveling further down the electrode array towards the distal end. By establishing a known volume and/or surface area that will be saturated, the amount of drug flow per unit time into the cochlea can be estimated or otherwise known.

It is also noted that in an exemplary embodiment, a sheath or the like can be placed around the electrode array so that even if the underlying body is saturated with drug, the drug cannot elute from the surface of the carrier because of the sheath. Indeed, in an exemplary embodiment, the sheath can be utilized instead of or in addition to the barrier 1310. A sheath upstream from the opening 1150 can be located about the electrode array, and/or a sheath downstream from the opening 1150 can be located about the electrode array. By providing openings in the sheath, which openings have spacing matching the electrodes, the electrodes can be utilized with the sheath in place. The limited amount of drug that will elute through the openings around the electrodes could be minimal relative to that which would otherwise be the case in the absence of the sheath. By way of example, the sheath could allow current flow but resistant to fluid flow. In an exemplary embodiment, the sheath is made of ePTFE. In an exemplary embodiment, the sheath extends over the electrodes, and the current can flow through the sheath.

Still further, in an exemplary embodiment, the makeup of the silicone body at different locations can be different, so as to control the amount of therapeutic substance that will elute from the electrode array. By way of example only and not by way of limitation, the carrier body can have a first property within, for example, two or three or 4 mm on either side of the opening 1150, or some other arrangement, with respect to the longitudinal axis, and can have a second property for locations beyond that area. The first property is a property that allows the drug to easily pass therethrough, at least relative to the material having the second property, which does not allow the drug to easily pass therethrough. Thus, the drug will move from the carrier body into the cochlea at the locations where the material has the first property, and will not do so or at least substantially not do so at the other locations.

In view of the above, it is to be understood that in an exemplary embodiment, the electrode array has a passageway therethrough extending long at least a substantial portion of the electrode array. The reservoir is in fluid communication with the passageway (which includes embodiments with or without a valve, such as a valve that can completely stop the flow of therapeutic substance from the reservoir into the distal ends of the array), the drug flows from the reservoir to the passageway, and from the passageway, the drug enters the silicone body and then elutes therefrom. That said, it is noted that in at least some embodiments, the elution feature is not utilized, and the drug can be directly transmitted from the passageway into the cochlea, such as via orifices extending from the passageway to the surface of the electrode array.

Thus, in an exemplary embodiment, there is an apparatus, comprising an implantable electrode array including a plurality of electrodes supported by a silicone body, wherein the silicone body is a drug elutor and the implantable electrode array is configured for recharging of the silicone body with drug while the silicone body is implanted in a cochlea of a recipient. In some embodiments, the implantable electrode array includes a passage from the silicone body to a location proximal from the silicone body, and the apparatus is configured such that the passage provides a conduit for drug to be delivered to the silicone body to recharge the silicone body. Concomitant with the embodiments above, the apparatus includes an implantable reservoir that is in fluid communication with an interior of the silicone body and the apparatus is configured such that the reservoir passively recharges the silicone body. That said, in some alternate embodiments, a pump or the like can be utilized to actively recharge the silicone body.

In some embodiments, the teachings detailed herein enable an apparatus that is configured to deliver, on a first temporal period average, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 35, 40, 45, 50, 55, or 60 micrograms or any value or range of values therebetween in 0.1 microgram increments of therapeutic substance during a first temporal period over a second temporal period without recharging. In an exemplary embodiment, the first temporal period is 1, 2, 3, 4, 5, 6, 7 (i.e., a week), 8, 9, 10, 11, 12, 13, 14, 15, 16, 17, 18, 19, 20, 21, 22, 23, 24, 25, 26, 27, 28, 29, 30, 35, 40, 45, 50, 55, or 60 days, or any value or range of values therebetween in one day increments, and the second temporal period is 14, 15, 16, 17, 18, 19, 20, 21, 22, 23, 24, 25, 26, 27, 28, 29, 30, 35, 40, 45, 50, 55, 60, 65, 70, 80, 90, 100, 125, 150, 175, 200, 250, 300, 350, 365 (i.e., one year), 400, 500, 600, 700, 800, 900, or 1000 days or any value or range of values therebetween in one day increments.

Also, in some embodiments, as detailed above and further below, the apparatus is configured such that the reservoir can be refilled without surgery.

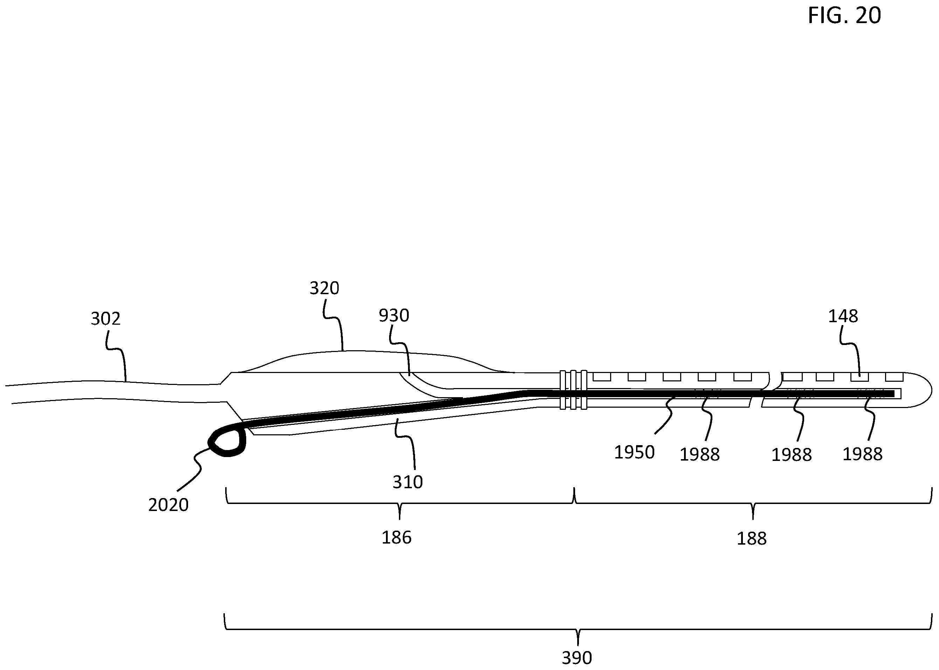

FIG. 19 presents an exemplary embodiment that utilizes the passageway in combination with a stylet passageway. Here, in this exemplary embodiment, there is a passage 1950, which passage forms a dual use passage as a stylet passageway and a drug delivery passageway. As can be seen, the passageway branches off into two separate directions. One direction goes to the reservoir 320 via 930, and the other is a passageway 1919 that extends towards the end of section 186. In an exemplary embodiment, as is seen in figure 20, a stylet 2020 is located in the stylet passageway 1919 and the drug deliver passageway / stylet passageway 1950, to provide for stiffening of the electrode array, which can be utilitarian by way of example only and not by way of limitation with respect to the electrode array that is a curved electrode array, where the stylet holds the electrode array relatively straight or otherwise straighter than that would be the case in the absence of the location of the stylet, so that the electrode array can be inserted into the cochlea, and the stylet is removed during insertion and/or after insertion. The embodiment of figure 20 and figure 19 can be utilized with, by way of example only and not by way of limitation, modiolus hugging electrode arrays, or in some embodiments, mid-scala electrode arrays.

**In** exemplary embodiments, after the stylet is completely removed from the passageway, the end of the passageway 1925 is plugged with some form of plug (not shown), which will seal the passageway. The plug can be an extended plug which extends all the way along the length of the passageway 1919 to about the location of the juncture, while in other embodiments, the plug can be a plug in limited dimensions that really only closes the end of the passageway 1919. After the passageway is plugged, or otherwise closed (in some embodiments, a crimping device or the like can be utilized to simply crimp the end of the passageway closed - a heating device can be utilized to heat seal or heat shrink the passageway 1919 - an adhesive or a liquid substance that later hardens can be inserted into the passageway 1919 to close the passageway - any device, system, and/or method that can close the passageway 1919 to enable the teachings detailed herein can be utilized in at least some exemplary embodiments), the reservoir 320 is charged or otherwise filled, and drug delivery can be commenced. In an exemplary embodiment there is a plug that is delivered via the stylet as the stylet is withdrawn. In this regard, the stylet can be part of an assembly that drags or pushes the plug into the opening as the stylet is withdrawn. In an exemplary embodiment, the assembly can be configured such that the plug is biased to move into the opening, but is prevented when the stylet is present, but then moves to the opening once the stylet is not in the way. For example, a spring mechanism can be present that is under compression when the stylet is in the way of the plug (the plug pushes back on the spring), and then when the stylet is not in the way, the spring jams the plug into the opening.

Note also that in at least some exemplary embodiments, a seal or the like can be located within the passageway 1919 or at a location outside the passageway, which seals around the stylet, and thus preventing drug from flowing out of the passageway 1919 in the event that the reservoir 320 is filled while the stylet is located in the passageway 1919 and/or 1950. Here, the seal can seal around the stylet, and once the stylet is removed, can seal the passageway. Indeed, in some embodiments, this can delete any utilitarian value for a plug, although in other embodiments of plug can be used. Indeed a second time, the seal can be located at the end of the passageway 1925, and the plug can interface with the seal to as to close the passageway 1919 in a manner that the stylet close the passageway when located therein. Still, it is noted that in at least some embodiments, the plug is not necessary or otherwise not utilized - the seal completely seals / effectively seals the passageway 1919 upon complete removal of the stylet.

Thus, as can be seen, in an exemplary embodiment, the electrode array is a cochlear implant electrode array, and the passageway is a lumen for a stylet of a cochlear implant curved electrode array. In an exemplary embodiment, the lumen for the stylet can be filled with therapeutic substance, and the array assembly is configured to enable the therapeutic substance to permeate into the silicone, and thus elute from the silicone into the cochlea.

In an exemplary embodiment, the lumen for the stylet can be used to achieve a functionality corresponding to a reservoir (as with the passageway, it is not a reservoir as that term is used herein). By way of example only and not by way of limitation, after the stylet is removed, the lumen can be provided with a therapeutic substance, such as via the utilization of a needle or the like or some other device, and filled with therapeutic substance. After such, the lumen can be plugged. In an exemplary embodiment, periodically, the lumen can be recharged with therapeutic substance. By way of example only and not by way of limitation, in an exemplary embodiment, a needle can be inserted into the plug / through the plug, where the plug is made out of a material that will permit passage of the needle therethrough, but then will immediately seal or subsequently seal after the needle is withdrawn, and then the needle can be used to inject therapy took substance into the lumen to recharge the lumen. This can be executed periodically or as needed. In another exemplary embodiment, a conduit or the like extends from the lumen to a location that is more easily accessed by a healthcare professional or the like, such as a location beneath the skin, where the healthcare professional can insert a needle through the skin and to the conduit so as to recharge or otherwise refill the lumen. In another exemplary embodiment, this conduit can be connected to any of the refill ports detailed herein.

Note that in an exemplary embodiment, there are sensors or the like that are included with the reservoir or otherwise with the electrode array assembly that can enable sensation or otherwise an evaluation of the amount of therapeutic substance that remains in the reservoir or otherwise in the electrode array assembly. In this regard, in an exemplary embodiment, the electrode array assembly can be configured to communicate a signal indicative or otherwise based on the level of the amount of therapeutic substance that can be communicated from the receiver stimulator to the external component, thus giving an indication or otherwise enabling an indication of how much therapeutic substance remains or otherwise of any therapeutic substance remains, etc.

Returning back to figure 19, it can be seen that there are sections 1988 along the passageway 1950. These are sections where the passageway 1950 or otherwise the walls forming the passageway are porous at some sections along the length of the passageway, and not other sections, although in other embodiments, the sections can extend a substantial length along the passageway. Here, instead of the drug exiting out of an open end of the passageway (here, there is no open end - this having utilitarian value with respect to providing a configuration where the end of the passageway abuts the end of the stylet, and thus prevents the stylet from possibly pushing through the silicone body), the drug exits the passageway through the porous sections 1988. It is noted that this embodiment can be combined with other embodiments where there is an open end of the passageway as well.

In view of the above, it is to be understood that in at least some exemplary embodiments, the reservoir is under pressure such that the drug is forced from the reservoir into the carrier body and/or the silicone body substantially limits the flow of drug out of the drug reservoir.

In an exemplary embodiment, the pressure under which the drug is located is a pressure that is at least 1.1, 1.2, 1.3, 1.4, 1.5, 1,6, 1.7, 1.8, 1.9, 2.0, 2.25, 2.5, 2.75, 3.0. 3.25, 3.5, 3.75, 4.0, 4.5, 5, 5.5, 6, 6.5, 7, 7.5, 8, 8.5, 9, 9.5, 10, 11, 12, 13, 14, 15, 16, 17,18, 19, 20, 21, 22, 23, 24, 25, 30, 35, 40, 45, 50, 55, 60, 65, 70, 75, 80, 85, 90, 95, 100 or more times greater than the ambient pressure inside the cochlea, inside the middle ear, and/or the statistical average atmospheric pressure at sea level in Washington, D.C. for the calendar year 2015 based on data at Dulles Airport and/or 1 atmosphere or any value or range of values therebetween in increments of 0.01 times (1.33 times, 15.15 times, 2.27 to 44.44 times, etc.), all other things being equal.

Also, as can be seen from the above, in at least some exemplary embodiments, the electrode array is configured such that the drug elutes from the array upon entering the array into the cochlea. In an exemplary embodiment, the rate of elution is at least 1.1, 1.2, 1.3, 1.4, 1.5, 1,6, 1.7, 1.8, 1.9, 2.0, 2.25, 2.5, 2.75, 3.0. 3.25, 3.5, 3.75, 4.0, 4.5, 5, 5.5, 6, 6.5, 7, 7.5, 8, 8.5, 9, 9.5, 10, 11, 12, 13, 14, 15, 16, 17,18, 19, 20, 21, 22, 23, 24, 25, 30, 35, 40, 45, 50, 55, 60, 65, 70, 75, 80, 85, 90, 95, 100, 125, 150, 175, 200, 250, 300, 350, 400, 450, 500, 600, 700, 800, 900, 1000, 1250, 1500, 1750, 2000, 2500, 3000, 3500, 4000, 5000, 6000, 7000, 8000, 9000, or 1000 or more times less than that which would otherwise be the case in the absence all that the body of the array limiting the rate of flow of the drug into the cochlea or any value or range of values therebetween in increments of 0.01 times (1.33 times, 15.15 times, 2.27 to 44.44 times, etc.), all other things being equal. Note also that in at least some exemplary embodiments, the aforementioned rates are also applicable for the embodiments that utilize the flow restrictor. Note further that in at least some exemplary embodiments, the aforementioned rates are also applicable for the embodiments that utilize alternate opening and closing of the valve, over a period of time, such as any of the temporal periods detailed herein (e.g., 400 times less drug enters the cochlea over a period of 6 months than that which would have been the case in the absence of the valve).

In view of the above, it can be seen that in some exemplary embodiments, there is an apparatus comprising an implantable drug reservoir, and an implantable electrode array including a plurality of electrodes. The apparatus further includes a drug elutor in fluid communication with the drug reservoir, which drug elutor is attached to the electrode array. In this embodiment, the implantable drug reservoir is part of an assembly that also includes the electrode array. In an exemplary embodiment, the array and the reservoir are unitized, as noted above.

In an exemplary embodiment, concomitant with the teachings above, the assembly is an electrode array assembly. Further, the implantable drug reservoir is carried by the electrode array. In an alternate embodiment, the implantable drug reservoir is not carried by the electrode array. Also, in some embodiments, as noted above, the electrode array assembly is a cochlear implant electrode array assembly, and the drug reservoir is configured to be located in a middle ear cavity when the electrode array is fully implanted in a cochlea of a recipient. Note also that in some embodiments, the electrode array can be fully implanted without being fully inserted. In this regard, there exists the so-called or sometimes referred to as the "partially inserted" array, where the electrode array is inserted for may be the first 9 of 22 electrodes, because the recipient retains residual hearing in the lower frequencies, and thus it is not entirely utilitarian to have the electrode array inserted into the locations of the cochlea associated with the residual hearing of the recipient. Accordingly, a fully implanted electrode array is an implanted electrode array that corresponds to that which would result at the end of the surgery / would be the positional state of the electrode array after the recipient is "sewn up." That said, in most instances, a fully implanted electrode array will be a fully inserted electrode array. In any event, any disclosure herein of full implantation corresponds to a disclosure of a fully inserted electrode array in some embodiments, and disclosure of a partially inserted electrode array in some other embodiments, unless otherwise noted.

Some embodiments of the drug elutor have been described above. In in some embodiments, the drug elutor has a porosity of 1, 2, 3, 4, 5, 6, 7, 8, 8, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, or 65 micrometers or any value or range of values therebetween in 0.1 micrometer increments.

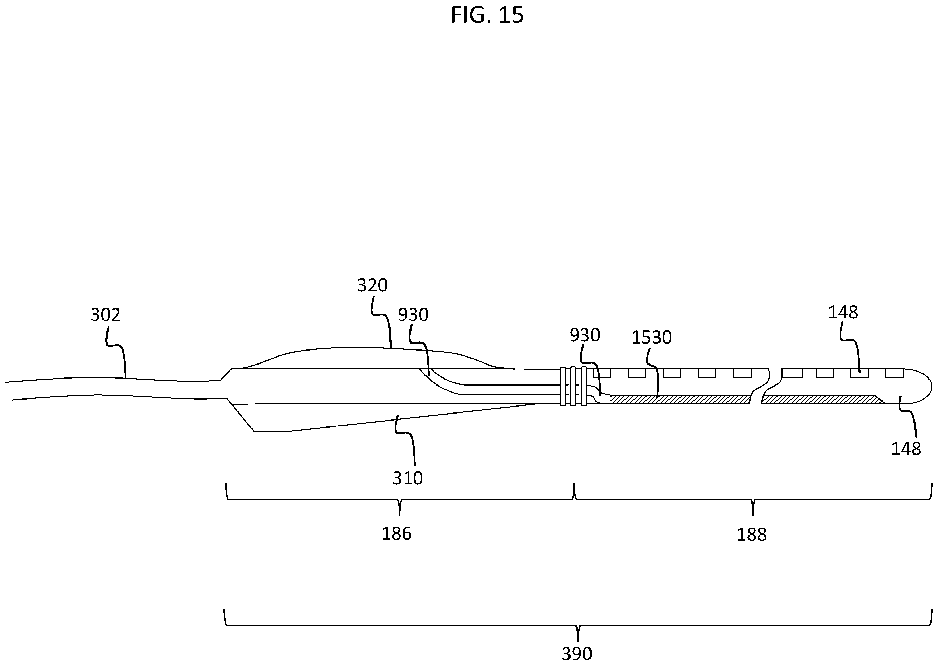

In some embodiments, the drug elutor is a backstrap of a cochlear electrode array, while in other embodiments, it is not such a backstrap. FIG. 15 presents an exemplary embodiment that utilizes a backstrap 1530, which backstrap is in fluid communication with the passageway 930, which passageway is in fluid communication with the channel 320 as seen. Figure 15 depicts the backstrap 1530 extending along almost all of the length of the electrode array, while figure 16 depicts the backstrap 1630 extending along only a distal portion of the electrode array. It is noted that while the embodiments depicted in the figures present a contiguous backstrap, in alternate embodiments, two or more backstrap can be utilized, separated by spacing. Note further that while the embodiments depicted in figures 15 and 16 depict the passageway 930 meeting the backstrap at the proximal end of the backstrap, in some alternate embodiments, the passageway 930 interfaces with the backstrap at the middle portion and/or a more distal portion. Also, in some exemplary embodiments, the passageway 930 can interface with the backstrap at a plurality of locations. By way of example only and not by way of limitation, in an exemplary embodiment, the passageway can extend in parallel along some of the length of the backstrap, and can have two or more ports extending from the passageway to the backstrap, each individually supplying drug to the backstrap at those locations, such that the backstrap is "charged" at different locations. Such can have utilitarian value with respect to more evenly distributing the drug along the backstrap. In an exemplary embodiment, the passageway can be continuous with the blackstrap. In an exemplary embodiment, the passageway effectively continuously provides therapeutic substance to the blackstrap along the length thereof.

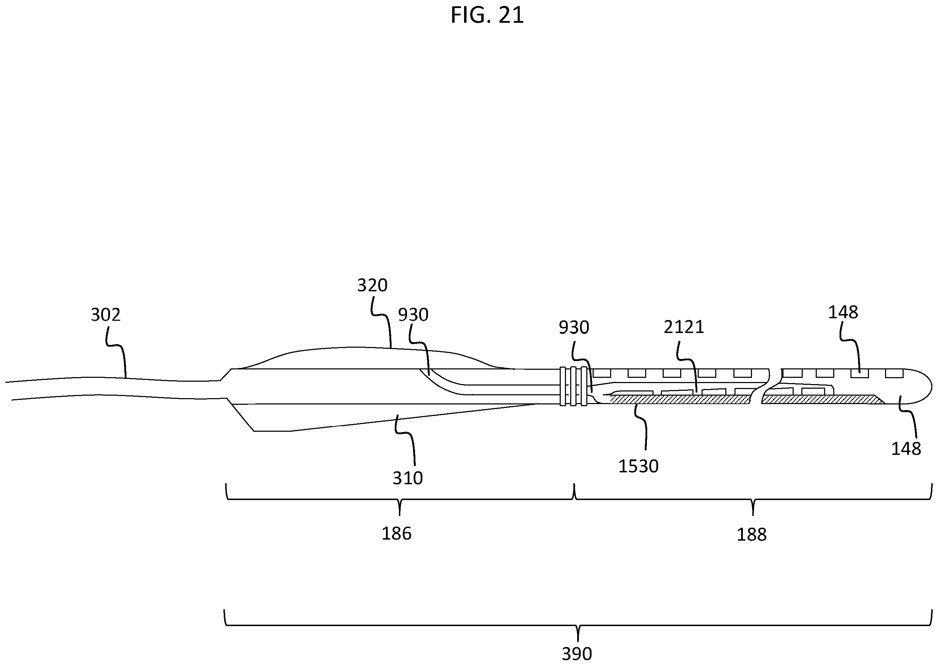

Note also that in at least some exemplary embodiments, one or more or all of the aforementioned passageways of the plurality of passageways can have separate valves, such that different sections of the backstrap can be charged in a controllable manner or otherwise not charged in a controllable manner. Note also that in an exemplary embodiment, this feature with a plurality of valves can be utilized with respect to other embodiments, such as where the electrode array body/carrier body is the component that elutes the drugs. Figure 21 presents such an exemplary embodiment, where the passageway 930 extends along much of the length of the backstrap 1530, and passageways 2121 are located along the length of that extension. In this regard, in an exemplary embodiment, the passageway can have a plurality of openings, one or more or all of those plurality of openings having separate valves, so that different sections of the body can be charged and/or prevented from being charged with drugs at different temporal periods, etc.

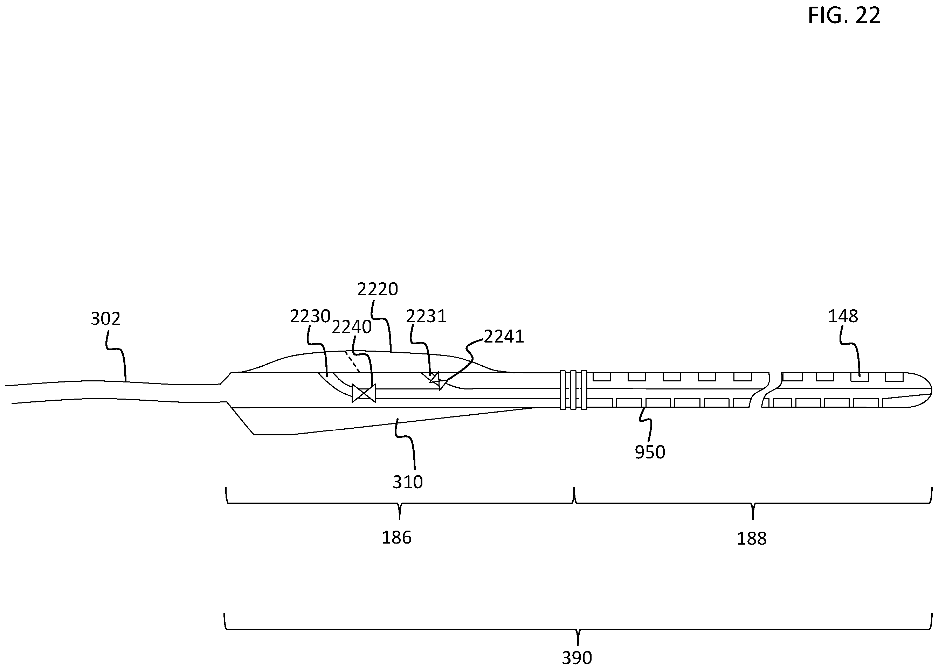

It is also noted that while the embodiments detailed above have been directed towards a single reservoir that delivers a single drug or otherwise a single therapeutic substance, in an alternative embodiment, there can be two or more reservoirs and/or the reservoirs can be bifurcated or trifurcated or otherwise divided so as to contain separate therapeutic substances. In this regard, in an exemplary embodiment, a plurality of passageways can extend to separate reservoirs and/or the divided reservoir. In an exemplary embodiment of this is depicted in figure 22. Here, passage 2230 and passage 2231 respectively extend to separate portions of reservoir 2220, each portion being separate from the other by a membrane represented by the dashed line, and containing a different therapeutic substance. Here, separate valves 2240 and 2241 control the flow of the therapeutic substance from passages 2230 and 2231, respectively, to the main passage. By controlling the valves, delivery of one therapeutic substance versus the other therapeutic substance that temporally separate locations, and/or delivery of separate therapeutic substances at the same time, can be achieved. It is also noted that while the single main passageway is presented in this embodiment, in an alternate embodiment, the passageways can be completely separate so that the drugs or otherwise therapeutic substances will never contact one another, even in a residue form, until they are distributed into the cochlea. Note also that in some embodiments, instead of the valves, flow restrictors can be used, etc. Any arrangement that can enable the delivery of two or more separate therapeutic substances according to the teachings detailed herein can be utilized in at least some embodiments. Note further that while the embodiment of figure 22 presents a reservoir that is divided, in alternate embodiments, two or more separate reservoirs can be utilized, with each passage to 2230 and 2231 leading to the separate reservoirs.

FIG. 15 presents an exemplary embodiment that utilizes a backstrap 1530, which backstrap is in fluid communication with the passageway 930, which passageway is in fluid communication with the channel 320 as seen. In this regard, backstrap 1530 is configured so as to, when the electrode array includes a slot, for example, which can be provided for a matched-in-shape drug releasing device, such as the backstrap, such backstrap can be utilized to deliver drug to the cochlea, at least when the backstrap is in fluid communication with the reservoir. In various embodiments, the drug eluting portion may be a layer of material sandwiched between two layers of non-drug eluting material. For example, the drug eluting portion may constitute 0.25 to 2% of the mass of the electrode array. The drug eluting portion may be embedded within non-drug eluting material so that the thickness of the non-drug eluting material determines the rate at which the pharmaceutical agent will be released. The drug eluting portion may begin, by way of example only and not by way of limitation, at 3 mm or less from where the electrode array enters the inner ear. The release rate of the pharmaceutical agent may be determined by one or more of the crosslink density of the material in the drug eluting and non-drug eluting portion, the amount of surface area of the drug eluting portion which is exposed to the non-drug eluting sandwich, and the volume of the drug eluting portion.

In some embodiments, the drug eluting portion may include first and second drug eluting portions, each portion adapted to release a different pharmaceutical agent. The electrode array may include multiple electrical contacts for electrically stimulating the cochlear tissue, at least one of the contacts being coated with the pharmaceutical agent. The pharmaceutical agent may be in the form of solid particles of less than 100 µm mixed into the material of the drug eluting portion. The release rate of the pharmaceutical agent may be based on having particles of the pharmaceutical agent in a plurality of defined sizes. For example, at least 90% of the particles maybe less than 50 µm, and/or at least 50% of the particles maybe less than 10 µm. The pharmaceutical agent may be a corticosteroid such as betamethasone, clobetasol, diflorasone, fluocinolone, triamcinolone, salt, ester, or combination thereof. Or, the corticosteroid maybe dexamethasone, for example, the electrode array maybe adapted to release between 0.1 µg and 1 µg of dexamethasone during an initial 24-hour period of use. In some embodiments, the pharmaceutical agent may be an anti-inflammatory agent. For example, the saturated solubility in normal saline of the anti-inflammatory agent may be not less than 80 µg/ml at 37° C. The electrode array may be adapted to release between 1 µg and 5 µg of anti-inflammatory agent during the first week after implantation. The pharmaceutical agent could be an antibiotic, antioxidant or growth factor.

FIGs. 15 and 16 show examples of cochlear implant electrode arrays structured so as to include a drug eluting portion in the form of a backstrap 1530 / 1630, and a non-drug eluting portion (the remainder of the array in some embodiments). In each of the examples shown in FIGs. 15 and 16, the cross-hatched region represents material adapted to release pharmacological agent, i.e., the drug eluting portion / backstrap. The unshaded regions in the figures represent material without drug eluting functionality, i.e., the non-drug eluting portions.

A cross-section of the electrode array may typically be elliptical or oval in shape. The figures depict an embodiment in which the lower half of a portion of the electrode array includes is the drug eluting portion including drug eluting material which, in some embodiments, time releases a pharmacological agent to the surrounding fluid of the inner ear. The upper half of this embodiment is the non-drug eluting portion containing material without drug eluting functionality. As noted above, in some exemplary embodiments, there can be two or more different drug elutors. In an exemplary embodiment, the backstrap is implemented in accordance with any of the teachings of US patent application publication number 2007/0213799 to Jolly, published on September 13, 2007, by the USPTO.

Thus, in an exemplary embodiment, there can be two or more different drug eluting portions, each of which may be adapted to release a different pharmacological agent.

In an exemplary embodiment, the drug eluting portion includes the entire lower half of the intracochlear portion of the electrode array, while in other embodiments, the drug eluting portion extends along more than or less than or equal to 90, 85, 80, 85, 70, 65, 60, 55, 50, 45, 40, 35, 30, 25, 20, 15, or 10% of the length of the intracochlear electrode array portion, or any value or range of values therebetween in 1% increments.

In an exemplary embodiment, there is drug release along the entire length of the array, while in other embodiments, the drug release occurs only along a portion of the array.

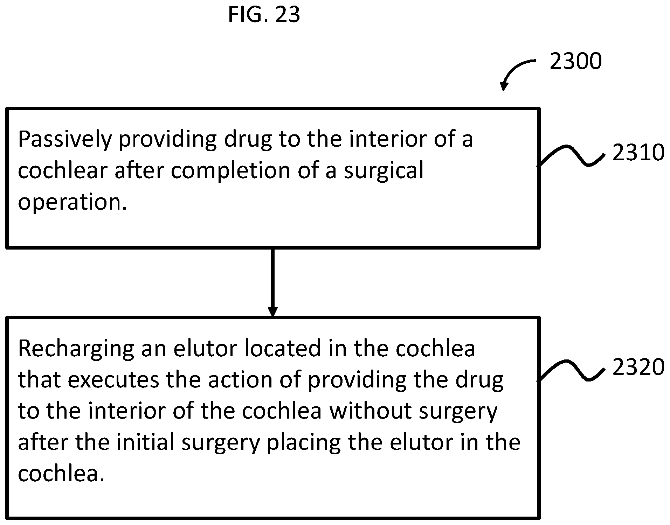

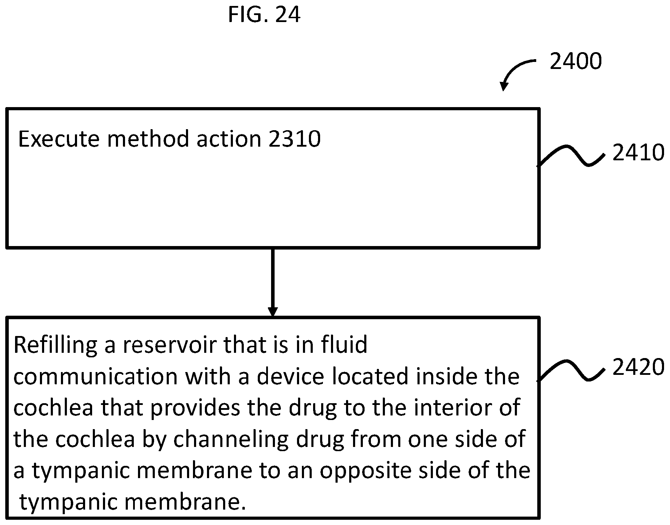

In connection with Figs. 23 and 24, not claimed methods shall be described for better understanding the function of the claimed apparatus. In this regard, a method includes the action of passively providing drug to the interior of a cochlear after completion of a surgical operation, wherein the action of passively providing drug is a result of a pressure gradient relative to an interior environment of the cochlea. In an exemplary method, the method further includes the action of recharging an elutor located in the cochlea that executes the action of providing the drug to the interior of the cochlea without surgery after the initial surgery placing the elutor in the cochlea. FIG. 23 presents an exemplary algorithm for an exemplary method, method 2300, which includes method action 2310, which corresponds to the first method action just detailed above, and method action 2320, which corresponds to the second method action just detailed above. In an exemplary method, the surgical operation is a cochlear implant electrode array implantation.

With respect to the phrase initial surgery, that constitutes the surgery that resulted in the elutor, which in the embodiment where the elutor is part of the electrode array assembly, also corresponds to the surgery that resulted in the electrode array assembly, being implanted into the recipient. Surgery is completed upon the "sewing up" of the opening that was made in the recipient to implant the elutor / array. In an exemplary method, the elutor and/or the electrode array are implanted inside the cochlea, and such can be reached only by cutting into the recipient. Note further that in some exemplary methods, surgery might be executed in a less invasive manner, such as by obtaining access to the cochlea through the outer ear and the middle ear. Such still constitutes surgery in that ultimately, an opening into the cochlea will be required to be made, at least in method associated with a cochlear implant. It is noted that surgery can be completed even though the recipient is still located in the operating room. In this regard, some exemplary methods can include the action of filling the reservoir or the like while the recipient is in the operating room, albeit the surgery is completed and the skin of the recipient is sewed up.

That said, it is noted that in some methods, the aforementioned reservoirs and the like are filled during the surgical operation. Still, methods according to the teachings detailed herein enable the filling and/or refilling of the reservoir post-surgery / without having to cut into skin of the recipient again.

In an exemplary embodiment, the aforementioned pressure gradient is passively maintained within a range of D% of the maximum pressure gradient over a period of at least K days. In an exemplary embodiment, K is 1, 2, 3, 4, 5, 6, 7 (i.e., a week), 8, 9, 10, 11, 12, 13, 14, 15, 16, 17, 18, 19, 20, 21, 22, 23, 24, 25, 26, 27, 28, 29, 30, 35, 40, 45, 50, 55, 60, 65, 70, 80, 90, 100, 125, 150, 175, 200, 250, 300, 350, 365 (i.e., one year), 400, 500, 600, 700, 800, 900, or 1000 or any value or range of values therebetween in 1 value increments.

In some embodiments, D is 10, 11, 12, 13, 14, 15, 16, 17, 18, 19, 20, 21, 22, 23, 24, 25, 26, 27, 28, 29, 30, 35, 40, 45, 50, 55, 60, 65, 70, 80, 90 or any value or range of values therebetween in 0.1% increments. By way of example only and not by way of limitation, in an exemplary embodiment, if the aforementioned pressure gradient is passively maintained within a range of 50% of the maximum pressure gradient over a period of at least a week, that means, whatever the maximum pressure gradient is in that week, the pressure gradient does not fall below 50% of that pressure gradient. In an exemplary embodiment, the aforementioned pressure gradient is passively maintained within a range of D% of a temporally based average maximum pressure gradient over a period of at least K days, where the temporally based average maximum pressure gradient is the average of maximums for at least 5, 10, 15, 20, 25, 30, 35, 40, 45, 50, 55, 60, 65, 70, 75, 80, 85, or 90 percent or any value or range of values therebetween in 0.1% increments of K. by way of example only and not by way of limitation, if the temporally based average maximum pressure gradient is the average maximums for at least 10% of K, where K is 7 days, then the maximum pressure gradients for a period of time corresponding to 10% of seven days would be averaged.

In an exemplary embodiment, the maximum pressure gradient is the maximum pressure gradient that exists after the reservoir is filled and the only drug or otherwise therapeutic substance that leaves the reservoir is that which is delivered to the recipient in general, and into the cochlea in particular.

In an exemplary embodiment, the aforementioned pressure gradient is passively maintained within a range of D% of the average pressure gradient for a period of 1, 2, 3, 4, 5, 6, 7, 8, 9, 10, 11, 12, 13, 14, 15, 16, 17, 18, 19, or 20 days or any value or range of values therebetween in 0.1 day increments that immediately preceded the temporal period made up of a period of at least K days.

In at least some embodiments, the action of passively providing drug to the interior of the cochlea occurs for at least H hours after K days from the date of the completion of a surgery which placed a drug delivering device into a recipient. Where H can be 50, 55, 60, 65, 70, 80, 90, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900, or 2000 or any value or range of values therebetween in 1 value increments.

In an exemplary embodiment, the action of passively providing drug to the interior of the cochlea occurs due to a stressed body that is elastically deformed (e.g., the reservoir / membrane of the reservoir 320).

In an exemplary embodiment, the drug delivery systems detailed herein are configured to provide an amount of therapeutic substance into the cochlea from the electrode array of 1, 2, 3, 4, 5, 6, 7, 8, 9, 10, 11, 12, 13, 14, 15, 16, 17, 18, 19, 20, 25, 30, 35, 40, 45, 50, 60, 70, 80, 80, 90, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900, or 2000 nanograms of material per day, or any value or range of values therebetween in 0.1 nanograms increments.

In an exemplary embodiment, the drug delivery systems detailed herein are configured to provide an amount of therapeutic substance into the cochlea from the electrode array of 1, 2, 3, 4, 5, 6, 7, 8, 9, 10, 11, 12, 13, 14, 15, 16, 17, 18, 19, 20, 25, 30, 35, 40, 45, 50, 60, 70, 80, 80, 90, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900, or 2000 micrograms of material per month, or any value or range of values therebetween in 0.1 nanograms increments. In an exemplary embodiment, the reservoir that is utilized to contain the therapeutic substance or otherwise store the therapeutic substance prior to delivery is configured to store sufficient amounts such that for at least one or more of the aforementioned delivery rates, the reservoir need not be refilled more than once in 20, 25, 30, 35, 40, 45, 50, 60, 70, 80, 80, 90, 100, 125, 150, 175, 200, 250, 300, 350, 400, 450, 500, 550, 600, 650, 700, 750, 800, 850, 900, 950, 1000, 1100, 1200, 1300, 1400, 1500, 1600, 1700, 1800, 1900, or 2000 days or any value or range of values therebetween in 1 value increments. In an exemplary embodiment, there is a drug delivery system that is configured to provide any value between and including 5 to 200 micrograms of therapeutic substance in a 2, 3, or 4-month period, without refilling.

An exemplary method includes providing any value between and including 5 to 200 micrograms of therapeutic substance in a 2, 3, or 4 month period, for a period of 1, 1.5, 2, 2.5, 3, 3.5, 4, 4.5, 5, 6, 7, 8, 9, 10, 11, or 12 years or more, and refilling 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, or 35 times during that time period.

FIG. 24 presents another exemplary algorithm for an exemplary not claimed method 2400, which is useful to understand the function of the claimed apparatus, which includes method action 2410, which includes executing method action 2310 detailed above. Method 2400 further includes method action 2420, which includes refilling a reservoir that is in fluid communication with a device located inside the cochlea that provides the drug to the interior of the cochlea by channeling drug from one side of a tympanic membrane to an opposite side of the tympanic membrane. In this regard, in an exemplary method, an opening is provided in the tympanic membrane, and a conduit extends from the reservoir to the opening, and a therapeutic substance can be directed into the conduit through the opening of the tympanic membrane, thus providing the therapeutic substance to the reservoir.

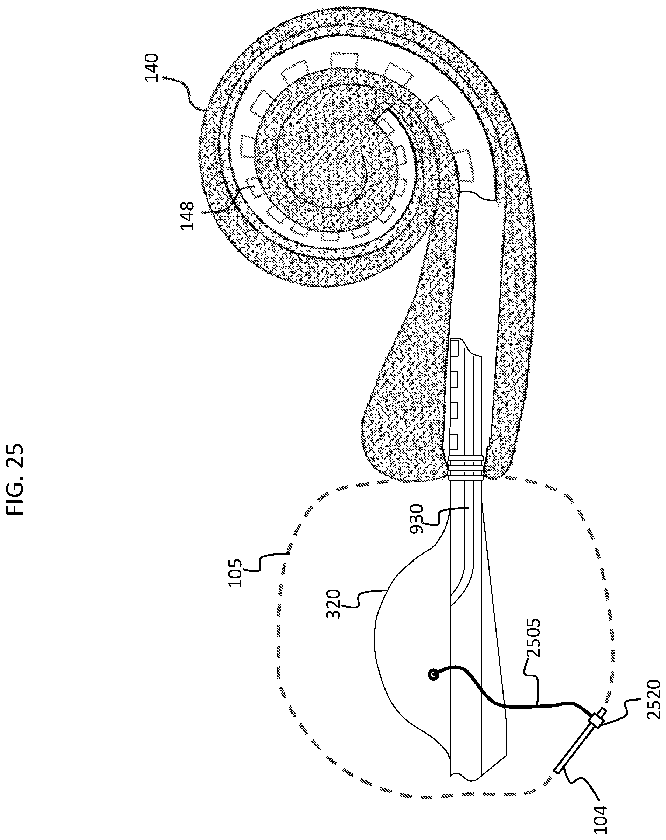

More to this, FIG. 25 presents an exemplary arrangement that enables such. More particularly, as can be seen, conduit 2505, which can be established by tube or the like, which can be a flexible or a rigid tube, extends from the reservoir 320 to a grommet 2520 that extends through the tympanic membrane 104. In an exemplary embodiment, the grommet 2520 is configured to receive a lumen, such as a needle or the like, so that a therapeutic substance can be driven from the outside of the recipient, through the conduit 2505, to the reservoir 320, thus filling the reservoir 320. In an exemplary embodiment, the conduit 2505 allows the re-pressurization of the reservoir 320. In an exemplary embodiment, the grommet 2520 includes a through hole through which the conduit 2505 can be reached. The grommet 2520 can include a self-sealing device that will enable a lumen to pass through, but will seal upon the removal of the lumen. In an exemplary embodiment, the grommet is a refill port that enables mechanical connection of a refill device to be connected thereto. In this exemplary embodiment, the only thing that "enters" the grommet is the therapeutic substance. In this regard, the grommet serves as a male component to which a female component of a refilling device is attached. That said, in some alternate embodiments, the reverse is the case.

In view of the above, in at least some exemplary embodiments, there is an apparatus as detailed herein that includes a conduit apparatus with a tympanic membrane grommet, the conduit apparatus extending from the grommet to the drug reservoir, wherein the grommet and the conduit apparatus enable refilling of the reservoir from the outer ear. Further, it can be seen that in an exemplary embodiment, there is an apparatus as detailed herein, wherein the grommet and the conduit apparatus enable pressurized refilling of the reservoir from the outer ear to repressurize the drug reservoir.

It is noted that in at least some exemplary embodiments, the grommet has utilitarian value with respect to enabling the eardrum to still function in substantially a normal manner, which includes a normal manner.

It is noted that in some alternate embodiments, conduit 2505 does not physically connect to the grommet. Instead, conduit 2505 can include a separate port that is located or otherwise spaced away from grommet 2520. In an exemplary embodiment, a lumen or the can be snaked through grommet 2520 to reach the port, and then via fluid coupling with the port, therapeutic substance can be provided to the port to refill the reservoir. This exemplary embodiment can have utilitarian value with respect to mechanically decoupling the conduit 2505 so that the conduit will not interfere with the movements of the tympanic membrane 104. In an exemplary embodiment, the port of the conduit 2505 can be hard mounted to a wall of the middle ear at a location adjacent the tympanic membrane, so that the conduit will not move or otherwise be displaced when the lumen is snaked to the conduit. Note further that in an exemplary embodiment, some embodiments do not utilize the grommet 2520. Instead, in an exemplary embodiment, as needed, a micro-needle or any needle or syringe element or lumen that can enable such or the like is inserted through the tympanic membrane and then to the port of the conduit 2505 which is located just behind the tympanic membrane, but at a distance where the tympanic membrane will not contact the port during normal function of the tympanic membrane. Indeed, in an exemplary embodiment, by positioning the conduit 2505 in a precise manner, a method can be enabled where the device that is utilized to refill the reservoir pushes the tympanic membrane inward an amount that is more than that which would exist during normal functioning of the tympanic membrane, but less than that which would damage the tympanic membrane, which pushing pushes the tympanic membrane against a faceplate or the like of the port of the conduit 2505, at which point the tympanic membrane can be punctured, where the back plate lessens the likelihood of damage to the tympanic membrane, and after the puncturing, the reservoir can be refilled by transferring therapeutic substance through the puncture into the port and into the conduit and thus the reservoir.

The above all said, in some alternate embodiments, there may not necessarily be a conduit 2505. In some embodiments, a lumen can be snaked from the tympanic membrane to the reservoir, where a port can be located. Further, embodiments include a trans-tympanic refill using a syringe to access the implant directly (e.g., at a proximal end of the array). In some embodiments, this is repeated at about 1 or 1.5, or 2 or 2.5 or 3 or 3.5 or 4 or 4.5 or 5 or 5o5 or 6 or more monthly intervals without significant risk to the tympanic membrane. Again, in some embodiments, grommet can be used to allow access to the middle ear without having to pierce the tympanic membrane.

In an exemplary embodiment, the needle, having pierced the tympanic membrane, is then used to access the reservoir directly, such as through a septum or even via a self-sealing reservoir. In this regard, in an exemplary embodiment, there is no conduit 2505. Indeed, in an exemplary embodiment, there can be no reservoir. In an exemplary embodiment, the needle is used to access the implant, such as at the extra-cochlear electrode array portion, to provide the therapeutic substance to the implant. In an exemplary embodiment, the extra-cochlear portion includes a septum or a self-sealing structure that leads to the passageway to the intracochlear portion. The needle is utilized to transfer therapeutic substance through the septum or otherwise to the structure to the passageway, thus refilling the implant.

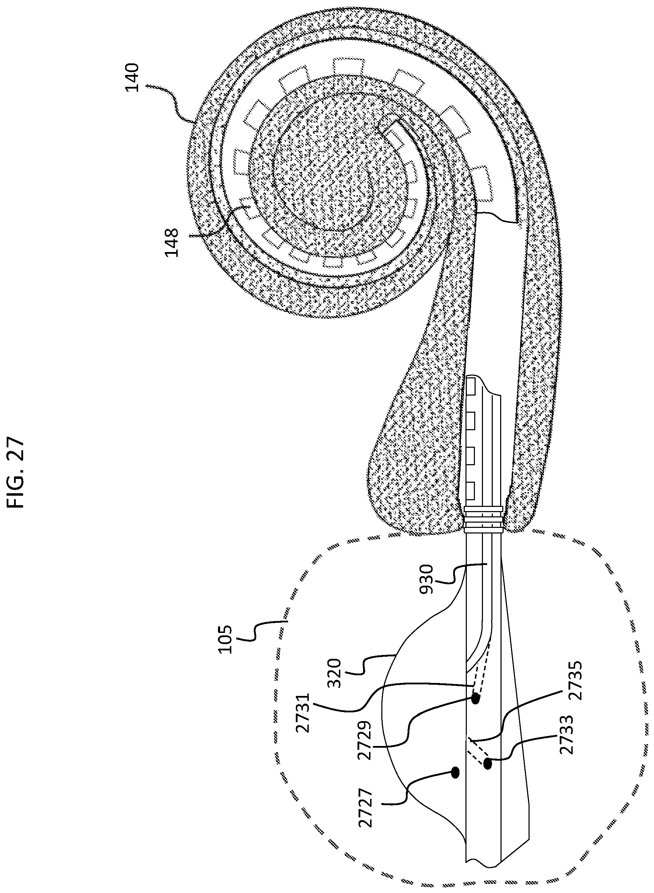

Figure 27 presents an exemplary embodiment according to the above teaching. Here, a septum 2727 is located in the side of the reservoir 320. In an exemplary embodiment, the septum is configured to enable a lumen of a needle to pass through in a hermetically sealed manner or otherwise in a effectively hermetically sealed manner, or any other manner that will avoid or otherwise reduce the possibility of contaminants entering the reservoir in general, and ultimately, the cochlea in particular, so that therapeutic substance can be delivered to the reservoir. Still, as noted above, in addition to this or alternatively, the reservoir can be made of a self-sealing material. Figure 27 also shows septum 2729 and passageway 2731, which leads to passageway 930. Passageway 2731 is shown in dashed lines to represent that this is an alternate embodiment, although in some embodiments, septum 2727 and 2729 can coexist. In this embodiment, the septum 2729 enables the lumen to reach passageway 2731 so that a needle can be utilized to charge or otherwise fill the system. In this embodiment, the passageway 930 can be utilized to "backfill" the reservoir 320. Still further, such a configuration can exist where there is no reservoir 320. Also, in some embodiments, a one-way valve can be positioned between passageway 2731 and the reservoir so as to avoid backfilling.

Figure 27 also shows septum 2733 and passageway 2735, which passageway leads to reservoir 320. This embodiment can have utilitarian value with respect to providing a more supportive structure for the septum 2733 relative to that which might be the case with respect to a septum that is located on the reservoir 320. In this regard, the extra-cochlear portion of the electrode array can provide resistance to the pushing action associated with moving the lumen through the septum, as opposed to the reservoir, which might just simply be pushed away from the tip of the lumen instead of having the lumen pierce the septum. Again, a one-way valve or the like can be utilized between the septum and the reservoir. Note also that the one-way valve can be utilized with septum 2727, where there is a pocket or the like inside of reservoir 320 that provides further isolation from the therapeutic substance inside reservoir 320 from the septum 2727.

Note further that in an exemplary embodiment, a guide or the like can be located proximate the septums. In an exemplary embodiment, a funnel like device can be located around septum 2733, for example, which will funnel the tip of the needle to the septum 2733. The funnel can be located at any of the refill ports according to the teachings detailed herein.

As can be seen, the septum can be incorporated directly into the reservoir or otherwise integrated with the reservoir. Here, this can be a resilient seal that can be repeatedly punctured by a syringe. In an exemplary embodiment, the septum can be punctured 2, 3, 4, 5, 6, 7, 8, 9, 10, 11, 12, 13, 14, 15, 16, 17, 18, 19, 20, 21, 22, 23, 24, 25, 26, 27, 28 29 or 30 or 40 or 50 or 60 or 70 or 80 or 90 or 100 or 150 or 200 times at least or more.

In any event, in at least some exemplary embodiments, the reservoir or the recharging port or the like can be accessed by/through the tympanic membrane and/or via the middle ear. In an exemplary embodiment, an endoscope or the like can be utilized to aid in the movements of the various components that are utilized to refill or otherwise recharge the reservoir.

It is also noted that in an exemplary embodiment, the reservoir itself can be of a configuration that extends to the tympanic membrane or close to the tympanic membrane or otherwise to the grommet. In an exemplary embodiment, at least a portion of the reservoir can be held to the wall of the middle ear at a location proximate the tympanic membrane, and/or can be attached to the grommet itself, where the grommet and/or the component that holds the reservoir against the middle ear wall home the reservoir irrespective of the deflation state of the reservoir. Thus, the reservoir can always be accessed at or proximate the tympanic membrane. Such can be accomplished by an elastomeric reservoir.

While the embodiments described above have typically been described with respect to the utilization of a port or the like, in some embodiments, self-sealing devices can be utilized (a septum, for example, as disclosed above). In this regard, in an exemplary embodiment, the reservoir can be structured so that a lumen can puncture the reservoir so as to refill the reservoir, and then upon removal of the lumen from the reservoir, the reservoir will self-seal.

While the embodiments described above have focused upon refilling through the tympanic membrane, it is noted that other embodiments enable refilling from other locations. By way of example only and not by way of limitation, in an exemplary embodiment, conduit 2505 can extend to a port that is located above the mastoid bone and behind and ear of the recipient. In an exemplary embodiment, a needle can be utilized that punctures the skin and reaches the port to the skin, and the needle is utilized to transfer therapeutic substance to the port, and then the needle is removed and the skin ultimately heals. Any arrangement that can enable refilling of the reservoir without another surgery the on that which resulted in the placement of the reservoir / drug delivery device can be utilized in at least some exemplary embodiments.

In view of the above, it can be seen that in at least some exemplary embodiments, there is a drug-eluting cochlear implant electrode array with drug in the silicone that does not have a finite lifespan vis-à-vis drug elution into the cochlea. In an exemplary embodiment, the therapeutic substance can be refilled / the drug eluting components can be recharged indefinitely (i.e., until the implanted device ultimately fails). In an exemplary embodiment, the therapeutic substance can be refilled/the drug eluting components can be recharged for as long as the device is functioning (i.e., until the device fails, where failure is not simply running out of drug). By utilizing the refillable reservoir, or even the non-refillable reservoir (more on this in a moment), the drug-eluting electrode array can be refilled or otherwise recharged.

As just noted, while many embodiments detailed herein have focused on a reservoir that is refillable, other embodiments do not utilize a refillable reservoir. That is, the reservoir is filled and it is not refilled. Indeed, in some embodiments, the reservoir is not refillable. Still, as will be understood, by utilizing the reservoir, the drug-eluting electrode array, or more accurately, the material that elutes the drug, can be recharged after drug has been eluted there from. This is contrasted to a non-rechargeable drug-eluting electrode array.

It is noted that while some exemplary embodiments are configured so that the silicone of the electrode array or otherwise the eluting component is charged with therapeutic substance prior to implantation into the cochlea, other embodiments are such that the silicone of the electrode array is not charged until after the electrode array is fully implanted into the cochlea. Note that these features can also be the case with respect to the other delivery systems disclosed herein.

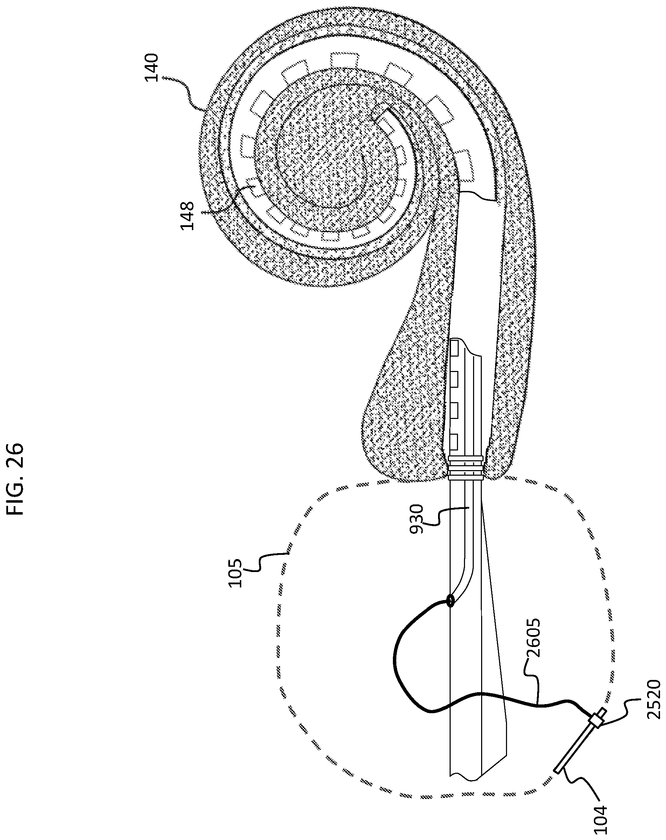

It is briefly noted that in at least some exemplary embodiments, an elutor can function as a reservoir in its own right, while in other embodiments, the elutor is a distinct and separate component from a reservoir and/or the passageway(s). Accordingly, the teachings detailed herein can utilize a non-eluting reservoir, such as reservoir 320. That said, in some alternate embodiments, reservoir 320 is not present. Instead, in an exemplary embodiment, the elutor is a device that provides the functionality of the reservoir. In this regard, by way of example only and not by way of limitation, there is an embodiment such as that seen in figure 26. Here, conduit 2605 corresponds to conduit 2505 detailed above, except that conduit 2605 extends to passage 930 instead to a reservoir. In this exemplary embodiment, conduit 2605 is utilized to recharge the elutor of the electrode array assembly, whether such is the carrier member / body of silicone that supports the electrodes and the leads of the electrode array, whether that is a separate such elutor, such as the backstrap elutor. In an exemplary embodiment, periodically or as needed, the grommet 2520 is interfaced with a refilling device in accordance with the teachings detailed above, so that the elutor can be recharged. This exemplary embodiment may require more frequent access / refilling than the embodiment of figure 25, all other things being equal. It is noted further that in at least some exemplary embodiments, passageways from the reservoir are distinct and separate components from the reservoir. In this regard, consistent with the teachings detailed herein, in an exemplary embodiment, the reservoir is located entirely outside of the cochlea when the electrode array is implanted in the recipient, irrespective of the fact that there is a passageway from the reservoir into the cochlea. Still, in some embodiments, the passageway can serve as a reservoir function in a sense, in that therapeutic substance can be stored therein. In any event, to be clear, the phrase "reservoir" is used in this application to encompass a structure that does not include the passageway.

In an exemplary embodiment, the apparatus is configured such that the silicone body can be recharged, no reservoir is located outside of the confines of the electrode array proper. Thus, by way of example only and not by way of limitation, referring to figure 19, there is no reservoir 320. The electrode array proper is section 188 and the body portions of section 186. In this regard, the stylet passage/lumen can be a reservoir. That said, in some embodiments, there is no passage within the electrode array, either.

Note also that while the embodiment of figure 26 is provided with the conduit 2605, in other embodiments, consistent with the embodiment of figure 25, the conduit is not present. Instead, a lumen or needle or the like is utilized to reach a port on the electrode array, and that is how the eluting substances recharged.

Any arrangement disclosed herein can be an arrangement that is refillable and/or rechargeable, unless otherwise specified.

While many of the embodiments focused above have been directed towards a reservoir that is an integral part of the electrode array assembly, where the reservoir is not a replaceable component of the electrode array assembly, in some alternate embodiments, the reservoir is a replaceable component of the electrode array assembly. By way of example only and not by way of limitation, in at least some exemplary embodiments, instead of refilling the reservoir, the reservoir is replaced with a new reservoir so as to replenish or otherwise recharge the implant with therapeutic substance. By way of example only and not by way of limitation, fluidic couplings can be utilized to couple and uncouple the reservoirs so as to place them into fluid communication with the remainder of the electrode array assembly. Accordingly, in at least some exemplary embodiments, there is a method that includes the action of replacing a reservoir with a new reservoir that is full of therapeutic substance.

Corollary to this is that in at least some exemplary embodiments, there are methods of converting existing or otherwise implanted medical devices that have drug delivery features to devices corresponding to the teachings detailed herein. By way of example only and not by way of limitation, there are existing cochlear implant electrode arrays that include drug reservoirs. Some embodiments include replacing those reservoirs with the reservoirs according the teachings detailed herein.

Note further that in at least some exemplary embodiments, existing electrode arrays that have never been used or otherwise have not been utilized for drug delivery are utilized for drug delivery. By way of example only and not by way of limitation, in at least some exemplary scenarios, there might be existing cochlear implant electrode arrays that have silicone carrier members that can be charged with therapeutic substance and utilized as elutors. In an exemplary embodiment, there is an exemplary scenario where a needle or the like is utilized to access the middle ear, and the needle is utilized to puncture or otherwise enter an extra-cochlear portion of the electrode array, and thus charge the silicone with the therapeutic substance. In this regard, in an exemplary embodiment, the nature otherwise the structure or makeup of the existing cochlear implant electrode array is such that the therapeutic substance will migrate from the extra-cochlear section to the intracochlear section. The silicone acting as an elutor.

Note also that in an exemplary embodiment, such as embodiments where there is a passageway/lumen for a stylet or the like, and existing implanted cochlear implant electrode array can be modified so that the reservoir is placed in fluid communication with the opening for the stylet. Thus, the stylet passageway can be utilized to transfer therapeutic substance into the cochlea, at least where the carrier member is utilized as an elutor. Corollary to this is that in some alternate embodiments, an extra reservoir is not added, but instead, a needle or some other device is utilized to place the therapeutic substance into the opening of the passageway for the stylet, and the needle is utilized to charge the carrier via introduction of the therapeutic substance into the passageway for the stylet.

It is noted that the just described embodiment is described in terms of an array that has been implanted in the recipient for days or weeks or months or even years. In an alternate embodiment, after the array has been inserted into the cochlea, but prior to the completion of the surgery, the reservoir is connected to the opening for the stylet. That is, because the stylet passageway is no longer needed as the stylet has been completely removed from the electrode array, and the array is inserted into the cochlea, the passage can then be repurposed for drug delivery by simply attaching a conduit to the opening. Thus, while the embodiments described in figures 19 and 20 depicted a separate conduit for the reservoir so as to reach the passageway for the stylet, in some alternate embodiments, a separate conduit extending from the reservoir can wrap around or otherwise extend around the array from the reservoir to be plugged into the opening for the stylet, thus eliminating the passage inside the electrode array from the reservoir to the passage 930.

While the above two paragraphs are focused on embodiments where the cochlear implant electrode array having the stylet passage was already implanted into the recipient, an alternate embodiment includes taking existing cochlear implant electrode arrays and attaching the reservoir to the opening for the stylet, thus converting stylet based arrays into drug delivery systems.

In an exemplary embodiment, the electrode array is a curved electrode array and the curvature of the electrode array reduces the amount of therapeutic substance eluted from the electrode array relative to that which would be the case if the electrode array was straight, all other things being equal. In an exemplary embodiment, the electrode array is a curved electrode array and the curvature of the electrode array increases the amount of therapeutic substance eluted from the electrode array relative to that which would be the case if the electrode array was straight, all other things being equal. (For example, if the therapeutic substance exists from the lateral side of the array, and the silicone of the lateral side stretches as the array curls, the substance delivery can be increased. All of this can be the opposite for compressed silicone, thus reducing the amount of substance eluted.)

It is noted that in at least some exemplary embodiments, the therapeutic substance located in the reservoir is a liquid-based therapeutic substance. In an exemplary embodiment, the therapeutic substance is devoid of any solid substances, including powdered substances. In an exemplary embodiment, the therapeutic substance contained in the reservoir or otherwise contained in the array is liquid and not solid/not a powder and/or not a salt based substance or the like. Further, the teachings detailed herein are directed towards, in at least some in exemplary embodiments, non-capsule based therapeutic substance delivery.

With respect to the embodiments disclosed herein that utilize an elutor, in at least some exemplary embodiments, the elutor is established and otherwise differentiated from other components of the electrode array assembly by the type of polymer, the molecular weight, the size, such as the cross-section of the elutor, the silica filler density, a prosody of the elutor, such as a micro / nano porosity, and/or a layered polymer structure. In this regard, the elutor can be a component that has any one or more of the properties just detailed that results in the component being an elutor and one or more the other components of the electrode array assembly can have one or more of the properties just detailed that results in that component not being an elutor.

In view of the above, it can be understood that in at least some exemplary embodiments, there is a passive pressurized delivery system configured to deliver the therapeutic substance directly into the cochlea, that combines the utilization of a flow restrictor and/or a valve so as to control the amount of therapeutic substance delivered in the cochlea.

It is noted that any disclosure with respect to one or more embodiments detailed herein can be practiced in combination with any other disclosure with respect to one or more other embodiments detailed herein. That is, some exemplary embodiments include any one or more of the teachings detailed herein combined with any one or more the other teachings detailed herein, unless otherwise stated such, providing that the art enables such. It is also noted that any disclosure herein of any feature corresponds to a disclosure of an exemplary embodiment that explicitly excludes that given feature from utilization with any one or more other features detailed herein unless otherwise specified providing that the art enables such.

It is noted that any disclosure herein of any method action corresponds to a disclosure of a device and/or system that enables that method action. It is noted that any disclosure herein of any method of manufacturing or otherwise developing or making a device disclosed herein corresponds to a disclosure of the resulting device that results from that method. It is noted that any disclosure herein of any apparatus and/or system corresponds to a disclosure of providing and/or making that apparatus and/or system. It is noted that any disclosure herein of any functionality corresponds to a device and/or system is configured to provide that functionality. It is noted that any disclosure of any device and/or system herein corresponds to a disclosure of a method of utilizing that device and/or system.

In this regard, it is noted that any disclosure of a device and/or system herein also corresponds to a disclosure of utilizing the device and/or system detailed herein, at least in a manner to exploit the functionality thereof. Further, it is noted that any disclosure of a method of manufacturing corresponds to a disclosure of a device and/or system resulting from that method of manufacturing. It is also noted that any disclosure of a device and/or system herein corresponds to a disclosure of manufacturing that device and/or system.

## Claims

1. An apparatus, comprising:
an implantable therapeutic substance reservoir (320); and
an implantable electrode array (188) including a plurality of electrodes (148) and
a silicone carrier body (146), wherein
the therapeutic substance reservoir (320) is in fluid communication with the silicone carrier body (146),
**characterized by**
a passageway (930) through the array (188) formed by a porous and/or an aerated silicone configured such that it enables the drug to pass between voids in said silicone such that the silicone carrier body (146) enables the therapeutic substance to elute through the silicone carrier body (146).

2. The apparatus of claim 1, wherein:
the reservoir is an elastomeric enclosure that expands upon the insertion of a liquid therapeutic substance therein, which expansion maintains the liquid therapeutic substance under pressure, and forces the therapeutic substance out of the reservoir and into the electrode array over time, and/or
the electrode array is configured such that the therapeutic substance elutes from the array upon entering the array into the cochlea, and/or the apparatus is pumpless, and/or
the apparatus is configured to enable refilling of the reservoir without explanting the reservoir.

3. The apparatus of claim 1, wherein:
the electrode array is configured so that the therapeutic substance passes through the material of the electrode array to leave the electrode array; and
the distance that the therapeutic substance passes through the material of the electrode array determines the rate of delivery of the therapeutic substance.

4. The apparatus of claim 1, wherein:
the electrode array is a cochlear implant electrode array.

5. The apparatus of claim 1, wherein:
the electrode array is a curved electrode array; and
the curvature of the electrode array increases the amount of therapeutic substance eluted from the electrode array relative to that which would be the case if the electrode array was straight, all other things being equal.

6. The apparatus of claim 1, wherein:
the apparatus is configured such that the reservoir maintains the carrier body loaded with therapeutic substance.

7. The apparatus of claim 1, wherein:
the silicone carrier body has a porosity of 1, 2, 3, 4, 5, 6, 7, 8, 8, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, or 65 micrometers or any value or range of values therebetween in 0.1 micrometer increments.

8. The apparatus of claim 1, wherein:
silicone body substantially limits the flow of therapeutic substance out of the therapeutic substance reservoir.

9. The apparatus according to claim 1, wherein the implantable therapeutic substance reservoir is part of an assembly that also includes the electrode array, wherein the implantable therapeutic substance reservoir is carried by the electrode array.

10. The apparatus of claim 9, wherein:
the electrode array assembly is a cochlear implant electrode array assembly; and the therapeutic substance reservoir is configured to be located in a middle ear cavity when the electrode array is fully implanted in a cochlea of a recipient.

11. The apparatus of claim 9, wherein:
the electrode array and the therapeutic substance reservoir are part of a single unit.

12. The apparatus of claim 1, wherein:
the silicone carrier body can be recharged; and
no reservoir is located outside of the confines of the electrode array proper.

13. The apparatus of claim 1, wherein:
the apparatus includes an implantable reservoir that is in fluid communication with an interior of the silicone carrier body; and
the apparatus is configured such that the reservoir passively recharges the silicone carrier body.

## Patentansprüche

1. Vorrichtung, umfassend:
ein implantierbares Reservoir (320) für therapeutische Substanzen; und
ein implantierbares Elektrodenarray (188) mit einer Vielzahl von Elektroden (148) und einem Silikonträgerkörper (146), wobei
das Reservoir (320) für therapeutische Substanzen in Fluidverbindung mit dem Silikonträgerkörper (146) steht,
**gekennzeichnet durch**
einen Durchgang (930) durch das Array (188), der durch ein poröses und/oder aufgeschäumtes Silikon gebildet ist, das so konfiguriert ist, dass es das Medikament zwischen Hohlräumen in dem Silikon hindurchströmen lässt, so dass der Silikonträgerkörper (146) die therapeutische Substanz durch den Silikonträgerkörper (146) hindurch eluieren lässt.

2. Vorrichtung nach Anspruch 1, wobei:
das Reservoir eine elastomere Umhüllung ist, die sich beim Einfüllen einer flüssigen therapeutischen Substanz darin ausdehnt, wobei diese Ausdehnung die flüssige therapeutische Substanz unter Druck hält und die therapeutische Substanz im Laufe der Zeit aus dem Reservoir in das Elektrodenarray drückt, und/oder
das Elektrodenarray so konfiguriert ist, dass die therapeutische Substanz beim Einsetzen in die Cochlea aus dem Array eluiert, und/oder
die Vorrichtung pumpenlos ist und/oder
die Vorrichtung so konfiguriert ist, dass das Reservoir ohne Explantation des Reservoirs nachgefüllt werden kann.

3. Vorrichtung nach Anspruch 1, wobei:
das Elektrodenarray so konfiguriert ist, dass die therapeutische Substanz durch das Material des Elektrodenarrays hindurchgeht, um das Elektrodenarray zu verlassen; und
die Strecke, die die therapeutische Substanz durch das Material des Elektrodenarrays zurücklegt, die Abgaberate der therapeutischen Substanz bestimmt.

4. Vorrichtung nach Anspruch 1, wobei:
das Elektrodenarray ein Cochlea-Implantat-Elektrodenarray ist.

5. Vorrichtung nach Anspruch 1, wobei:
das Elektrodenarray ein gekrümmtes Elektrodenarray ist; und
die Krümmung des Elektrodenarrays die Menge der aus dem Elektrodenarray eluierten therapeutischen Substanz im Vergleich zu dem Fall erhöht, in dem das Elektrodenarray gerade wäre, wobei alle anderen Faktoren gleich bleiben.

6. Vorrichtung nach Anspruch 1, wobei:
die Vorrichtung so konfiguriert ist, dass das Reservoir den mit therapeutischer Substanz beladenen Trägerkörper aufnimmt.

7. Vorrichtung nach Anspruch 1, wobei:
der Silikonträgerkörper eine Porosität von 1, 2, 3, 4, 5, 6, 7, 8, 8, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 oder 65 Mikrometer oder jeden Wert oder Wertebereich dazwischen in Schritten von 0,1 Mikrometern aufweist.

8. Vorrichtung nach Anspruch 1, wobei:
der Silikonkörper den Fluss der therapeutischen Substanz aus dem Reservoir für therapeutische Substanzen im Wesentlichen begrenzt.

9. Vorrichtung nach Anspruch 1, wobei das implantierbare Reservoir für therapeutische Substanzen Teil einer Anordnung ist, die auch das Elektrodenarray umfasst, wobei das implantierbare Reservoir für therapeutische Substanzen von dem Elektrodenarray getragen wird.

10. Vorrichtung nach Anspruch 9, wobei:
die Elektrodenarrayanordnung eine Cochlea-Implantat-Elektrodenarrayanordnung ist; und
das Reservoir für therapeutische Substanzen so konfiguriert ist, dass es sich in einer Mittelohrhöhle befindet, wenn das Elektrodenarray vollständig in einer Cochlea eines Empfängers implantiert ist.

11. Vorrichtung nach Anspruch 9, wobei:
das Elektrodenarray und das Reservoir für therapeutische Substanzen Teil einer einzigen Einheit sind.

12. Vorrichtung nach Anspruch 1, wobei:
der Silikonträgerkörper wiederaufladbar ist; und
sich kein Reservoir außerhalb der Grenzen des eigentlichen Elektrodenarrays befindet.

13. Vorrichtung nach Anspruch 1, wobei:
die Vorrichtung ein implantierbares Reservoir umfasst, das in Fluidverbindung mit einem Innenraum des Silikonträgerkörpers steht; und
die Vorrichtung so konfiguriert ist, dass das Reservoir den Silikonträgerkörper passiv wiederauflädt.

## Revendications

1. Appareil, comprenant :
un réservoir de substance thérapeutique implantable (320) ; et
un réseau d'électrodes implantable (188) comportant une pluralité d'électrodes (148) et un corps de support en silicone (146), dans lequel
le réservoir de substance thérapeutique (320) est en communication fluidique avec le corps de support en silicone (146),
**caractérisé par**
un passage (930) à travers le réseau (188) formé par une silicone poreuse et/ou aérée configuré de sorte qu'il permet au médicament de passer entre des vides dans ladite silicone, de sorte que le corps de support en silicone (146) permet à la substance thérapeutique de s'éluer à travers le corps de support en silicone (146).

2. Appareil selon la revendication 1, dans lequel :
le réservoir est une enceinte élastomère qui se dilate lors de l'insertion d'une substance thérapeutique liquide à l'intérieur, laquelle dilatation maintient la substance thérapeutique liquide sous pression, et force la substance thérapeutique hors du réservoir et à l'intérieur du réseau d'électrodes au cours du temps, et/ou
le réseau d'électrodes est configuré de sorte que la substance thérapeutique s'élue du réseau lors de l'entrée du réseau dans la cochlée, et/ou
l'appareil est dépourvu de pompe, et/ou
l'appareil est configuré pour permettre le réapprovisionnement du réservoir sans explantation du réservoir.

3. Appareil selon la revendication 1, dans lequel :
le réseau d'électrodes est configuré de sorte que la substance thérapeutique traverse le matériau du réseau d'électrodes pour quitter le réseau d'électrodes ; et
la distance sur laquelle la substance thérapeutique traverse le matériau du réseau d'électrodes détermine la vitesse de distribution de la substance thérapeutique.

4. Appareil selon la revendication 1, dans lequel :
le réseau d'électrodes est un réseau d'électrodes de type implant cochléaire.

5. Appareil selon la revendication 1, dans lequel :
le réseau d'électrodes est un réseau d'électrodes incurvé ; et
la courbure du réseau d'électrodes augmente la quantité de substance thérapeutique éluée du réseau d'électrodes par rapport à celle qu'elle pourrait être dans le cas où le réseau d'électrodes est rectiligne, toute les autres valeurs étant égales.

6. Appareil selon la revendication 1, dans lequel :
l'appareil est configuré de manière à ce que le réservoir maintient le corps de support chargé en substance thérapeutique.

7. Appareil selon la revendication 1, dans lequel :
le corps de support en silicone a une porosité de 1, 2, 3, 4, 5, 6, 7, 8, 8, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64 ou 65 micromètres, ou toute valeur ou plage de valeurs entre celles-ci par incréments de 0,1 micromètre.

8. Appareil selon la revendication 1, dans lequel :
le corps de silicone limite sensiblement l'écoulement de substance thérapeutique hors du réservoir de substance thérapeutique.

9. Appareil selon la revendication 1, dans lequel le réservoir de substance thérapeutique implantable fait partie d'un ensemble qui comporte également le réseau d'électrodes, dans lequel le réservoir de substance thérapeutique implantable est porté par le réseau d'électrodes.

10. Appareil selon la revendication 9, dans lequel :
l'ensemble de réseau d'électrodes est un ensemble de réseau d'électrodes de type implant cochléaire ; et le réservoir de substance thérapeutique est configuré pour être situé dans une cavité de l'oreille moyenne lorsque le réseau d'électrodes est entièrement implanté dans une cochlée d'un receveur.

11. Appareil selon la revendication 9, dans lequel :
le réseau d'électrodes et le réservoir de substance thérapeutique font partie d'une unité unique.

12. Appareil selon la revendication 1, dans lequel :
le corps de support en silicone peut être rechargé ; et
aucun réservoir n'est situé en dehors des limites du propre réseau d'électrodes.

13. Appareil selon la revendication 1, dans lequel :
l'appareil comporte un réservoir implantable qui est en communication fluidique avec un intérieur du corps de support en silicone ; et
l'appareil est configuré de sorte que le réservoir recharge passivement le corps de support en silicone.
